# EUROPEAN PATENT APPLICATION

(11) **EP 1 767 183 A1**
(43) Date of publication of application: **28.03.2007**
(21) Application number: 05751411.9
(22) Date of filing: 03.06.2005
(51) Int. Cl.: A61K 6/00

(54) **METHOD OF HAIR RESTORATION, METHOD OF FIXATION AND STABILIZATION OF HAIR FOUNDATION AGENT, AND HAIR RESTORATION SETTING AGENT USED IN THE METHODS**

(30) Priority: 14.06.2004 JP 2004205297
(71) Applicant: Shimizu, Yoshikazu, Nagaokakyo-shi Kyoto 6170836 (JP)
(72) Inventor: Shimizu, Yoshikazu, Nagaokakyo-shi Kyoto 6170836 (JP)
(74) Representative: Setna, Rohan P.
(86) International application number: PCT/JP2005/010277
(87) International publication number: WO 2005/120443

(57) **Abstract**

The present invention is a method for increasing hair volume, **characterized in that** a coating film with a finish in cylindrical or sheath form is formed on hair when a hair volume increasing agent is applied, fixed and stabilized on the surface of each hair in a portion where it is desired for the hair to increase in thickness so that a hair volume increasing coating film is formed, and a waterproof film coating agent is applied, fixed and stabilized on the surface of each hair on which said hair volume increasing coating film is formed.

## Description

### Technical Field

The present invention relates to a method for increasing hair volume according to which hair is artificially thickened. In addition, the invention relates to a method for fixing a foundation agent for hair which is used in the method for increasing hair volume. Furthermore, the invention relates to a hair volume increasing agent, a hair volume increasing agent set, a foundation agent for hair and a film coating agent which are used in these methods.

### Background Art

In order to gain the effects of increasing hair in a portion of a head having thin hair, natural or artificial hair has actually been implanted or has been attached using an appropriate means for attachment according to the prior art. The implementation of these means, however, requires treatment by a specialist as well as a large amount of time and cost, and thus, is very troublesome.

Hair loss at a late middle-age refers to a phenomenon where hair in the vicinity of the temples becomes thinner starting in around one's thirties, gradually followed by a decrease of the hair in the procephalic region and the occipital region, where hair has not actually fallen off but has slowly become thinner and shorter, and therefore, it can be said that the hair is becoming thinner rather than being lost. It is known that this is because the growth period during the hair cycle is shortened in such a manner that the growth stops while the hair is still short, and the hair which starts growing in the next growth period becomes thinner. Hair loss at a late middle-age is not limited to men, but hair thinning with the parietal region being the center progresses in middle-aged or older women, and it is estimated that the number of Japanese women who are troubled by hair thinning is approximately nine million.

It is considered that hair loss for women has recently been caused by an increase in the function of the androgenic hormones due to an imbalance of sex hormones within the body, and hair loss for both sexes is now referred to as hair loss at a late middle-age. That is to say, in many cases, hair loss at a late middle-age for both men and women, which appears during their thirties or later, is not the actual loss of hair, but the hair has gradually become thinner and shorter, making the hair volume less.

Therefore, the present inventor examined various researches in order to recover from this phenomenon of hair thinning, and as a result, invented a method for increasing hair volume by coating thinning hair with a thick film so that the hair was thickened by this coating film and the hair volume was increased. Furthermore, a hair volume increasing agent set, which is directly used for the implementation of this method for increasing hair volume, was also invented. Moreover, a method for fixing and stabilizing a foundation agent for hair, which is an important portion of the invention for increasing hair volume, as well as a foundation agent for hair and the like, which are directly used for the implementation of this method, were also invented.

As for one method which also intends to make thin hair look thick according to the prior art, Japanese Unexamined Patent Publication S62 (1987)-225263 shows that a short fiber material in powder form is used in order to gain the effects of increasing pseudo-hair, and an apparatus for spraying the powder is used to make this electrostatically adhere to hair and the like, and thereby, the effects of increasing hair volume can be gained. In the means shown in Japanese Unexamined Patent Publication S62 (1987)-225263, however, the state of the adhered powder is unstable and unreliable, and thus, the powder easily falls off after the application, and therefore, the effects of increasing hair volume are not practically sustained, and no practical effects of increasing hair volume can be gained.

Meanwhile, agents, which are referred to as hair volume increasing aerosols, in the form where volume increasing powder is added to a color spray for dyeing hair are also known as those shown in Japanese Unexamined Patent Publication H8 (1996)-143434 and Japanese Unexamined Patent Publication H9 (1997)-87146. These pseudo-hair volume increasing agents are products where the coloring effects and adhesion stability have been improved, though the effects of increasing hair volume are inferior in comparison with the effects of increasing hair volume which are gained by using a short fiber material and electrostatically attaching the powder through spraying. However, these cannot be categorized other than color sprays, which are hair dyeing agents for temporary coloring that also have effects of increasing hair volume. In addition, as for the method of usage, any of these is applied to the hair after doing the hair, and a fixing resin such as a hair spray, which is a stabilizing agent, is sprayed on top of the portion where the hair volume has increased, if necessary, so as to coat the portion where the hair volume has increased in an attempt to elongate the time, as much as possible, before this portion naturally falls off. These methods for stabilizing a powder or pigments are all temporary, and when it is hit by rain, it comes off and is washed away; when the hair after being dyed is touched, the dye or pigments come off and are transferred to another thing or another place; and it also comes off and is washed away when taking a bath, and therefore, it is necessary to prevent water from getting onto the hair when taking a bath.

As described above, the conventional methods for increasing hair volume are temporary methods for coloring hair and increasing hair volume through the application of a powder, except for the permanent method for increasing hair through the implantation or the grafting of facial hair or pseudo-hair. In the case of the latter, the patient has to be determined before attaching the facial hair or the pseudo-hair, and feels uneasy due to an unnatural feeling when facial hair is used or worries about feeling embarrassed in the case where the hair is detached, or the other people know the patient is wearing the facial or pseudo-hair. In the case of the former, there are problems

where it is troublesome for the user to apply it every day, the user worries about color loss during rainy weather, and in addition, the user cannot use a shower, take a bath or swim in a pool, and therefore, there are many situations such that the user has to pay careful attention, for example, in preventing water from getting onto the hair when using a public bath with companions during a trip.

### Disclosure of the Invention

### Problems to be Solved by the Invention

An object of the present invention is to develop an inexpensive and simple method for increasing hair volume, which does not have major defects as described above where the patient has to be determined to wear pseudo-hair, feels uneasy due to an unnatural feeling when facial hair is used, or worries about feeling embarrassed while wearing the facial or pseudo-hair, and which is a method for increasing hair volume while providing excellent coloring and volume to the hair with no falling off of the coloring pigments or the like. Another object of the invention is to additionally develop a hair volume increasing agent set which is directly used for the implementation of the method for increasing hair volume resulting from the above described object.

### Means for Solving the Problems

The present inventor performed diligent research, in view of the current situation as described above, in order to solve the above described problems, and as a result, found that a semi-permanent hair volume increasing agent could be gained by coating the surface of each hair with a component and the like that became a hair volume increasing agent as a waterproof coating film in cylindrical or sheath form, and thus, an item having effects of increasing hair volume, which never comes off from the hair due to the coating film in cylindrical form that is formed on the hair and has a cylinder length (length of the hair) that is considerably great relative to the cylindrical diameter (diameter of the hair) even when the respective strength of adhesion (connection) (between the hair and the hair volume increasing agent, between the hair and the foundation agent, between the foundation agent and the hair volume increasing agent, between the hair volume increasing agent and the film coating agent, and the like) becomes weak due to environmental factors and the passage of time, could be formed, and thereby, the present invention was completed.

The present invention includes the following (1) to (16).
(1) A method for increasing hair volume, characterized in that a hair volume increasing coating film with a finish in cylindrical or sheath form is formed on hair when a waterproof hair volume increasing agent is applied, fixed and stabilized on the surface of each hair in a portion where it is desired for the hair to increase in thickness.
(2) A method for increasing hair volume, characterized in that a coating film with a finish in cylindrical or sheath form is formed on hair when a hair volume increasing agent is applied, fixed and stabilized on the surface of each hair in a portion where it is desired for the hair to increase in thickness so that a hair volume increasing coating film is formed, and a waterproof film coating agent is applied, fixed and stabilized on the surface of each hair on which said hair volume increasing coating film is formed.
(3) The method for increasing hair volume according to the above described (2), wherein a waterproof hair volume increasing agent is used as the hair volume increasing agent.
(4) The method for increasing hair volume according to the above described (1) or (3), wherein the waterproof hair volume increasing agent for forming a waterproof hair volume increasing coating film is selected from among single,mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate, urethane modified resins, silicone modified resins.
(5) The method for increasing hair volume according to the above described (1), characterized in that a waterproof hair volume increasing agent in liquid form is applied on the surface of each hair, and after that, the applied waterproof hair volume increasing agent is fixed and dried while the hair is loosened using a comb or a brush, and thereby, a hair volume increasing coating film in cylindrical or sheath form is provided on each hair.
(6) The method for increasing hair volume according to any of the above described (2) to (5), characterized in that a waterproof film coating agent in liquid form is applied on the surface of each hair on which a hair volume increasing film is fixed and stabilized, and after that, the applied film coating agent is fixed and dried while the hair is loosened using a comb or a brush, and thereby, a waterproof coating film in cylindrical or sheath form is provided on each hair.
(7) The method for increasing hair volume according to the above described (4) or (6), characterized in that the waterproof film coating agent is an acid-proof, alkaline-proof and waterproof resin or a photo-setting resin.
(8) The method for increasing hair volume according to any of the above described (2) to (6), wherein the waterproof film coating agent is selected from among nitrocellulose, latexes which become a waterproof coating film after curing from among multipurpose latexes, such as NR or other general diene based synthetic polymers, polymer latexes, including MBR, BR, VP, NIR, SIR, IR, FKM, EPM and CSM,or single,mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate, urethane modified resins, silicone modified resins.
(9) The method for increasing hair volume according to any of the above described (1) to (8), characterized in that the resin of the hair volume increasing agent made of a waterproof resin in liquid form or the resin of the film coating agent made of a waterproof resin in liquid form is a waterproof and cleaning proof type resin having cleaning proof properties such that the coating film cannot be easily washed off with an acid or a neutralizing washing agent for hair after being dried in a non-neutralized state, and the waterproof and cleaning proof properties of the coating film can be reversed with an alkali neutralizing agent.
(10) The method for increasing hair volume according to any of the above described (1), (2), (3), (5), (6), (7) and (9), wherein the resin of the hair volume increasing agent made of a waterproof resin in liquid form or the resin of the film coating agent made of a waterproof resin in liquid form is a waterproof and cleaning proof type resin having cleaning proof properties such that the coating film cannot be easily washed off with an acid or a neutralizing washing agent for hair after being dried in a non-neutralized state, and the waterproof and cleaning proof properties of the coating film can be reversed with an alkali neutralizing agent,and the resin is any of non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate.
(11) The method for increasing hair volume according to any of the above described (1) to (10), characterized in that a foundation agent for hair is applied, fixed and stabilized on the hair before a hair volume increasing coating film is formed on the hair.
(12) A method for fixing and stabilizing a foundation agent for hair on hair before the hair is thickened, characterized in that a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied to all of the hair, and the hair is loosened with a comb or a brush until strands of hair to which the foundation agent for hair has been applied are separated from each other, the foundation agent for hair solidifies, making combing smooth, and thereby, a coating film that is compatible with hair coated with a foundation agent for hair in cylindrical or sheath form, has flexibility and is highly waterproof is formed on the surface of each hair.
(13) The method for fixing and stabilizing a foundation agent for hair according to the above described (12), wherein one component of the foundation for hair in liquid form that is compatible with hair, has flexibility, has little malleability and is highly waterproof is selected from single, mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl acrylamide acrylate, hydroxypropyl acrylate and butyl amino ethyl methacrylate, non-neutralizing polymers of carboxyl modified vinyl acetate, urethane resins, silicone resins, urethane modified resins, silicone modified resins.
(14) A method for increasing hair volume, characterized by comprising: the step of fixing and stabilizing a foundation agent for hair on hair before the hair is thickened, where a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied on the surface of the hair, and the hair is loosened with a comb or a brush until strands of hair to which the foundation agent for hair has been applied are separated from each other, the foundation agent solidifies, making combing smooth, and thereby, a coating film that is compatible with hair coated with a foundation agent for hair in cylindrical or sheath form, has flexibility and is highly waterproof is formed on the surface of each hair; and the step of repeating a hair volume increasing coating film forming operation until a desired amount of hair increase is gained, wherein in said hair volume increasing coating film forming operation, a hair volume increasing agent in liquid form for forming a hair volume increasing coating film having a large volume that has appropriate affinity and adhesiveness with a waterproof coating film is applied on the surface of the hair of the hair portion where said waterproof coating film is formed in such a manner that the surface of each hair is coated with a foundation agent for hair in cylindrical or sheath form in said step of fixing and stabilizing a foundation agent for hair on hair, hair is rubbed together and/or loosened using a comb or a brush so that the hair volume increasing agent is applied to the hair, and the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a foundation agent and a hair volume increasing agent in cylindrical or sheath form, and thus, the hair volume increasing agent is fixed and stabilized on the hair together with the foundation agent, said hair volume increasing agent in liquid form is applied on the surface of the hair on the hair portion where said hair volume increasing coating film is formed in order to further enhance the effects of increasing hair volume, the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a hair volume increasing agent in cylindrical or sheath form.
(15) A method for increasing hair volume, characterized by comprising: the step of fixing and stabilizing a foundation agent for hair on hair before the hair is thickened, where a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied on the surface of the hair, and the hair is loosened with a comb or a brush until strands of hair to which the foundation agent for hair has been applied are separated from each other, the foundation agent solidifies, making combing smooth, and thereby, a coating film that is compatible with hair coated with a foundation agent for hair in cylindrical or sheath form, has flexibility and is highly waterproof is formed on the surface of each hair; the step of repeating a hair volume increasing coating film forming operation until a desired amount of hair increase is gained, wherein in said hair volume increasing coating film forming operation, a hair volume increasing agent in liquid form for forming a hair volume increasing coating film having a large volume that has appropriate affinity and adhesiveness with a waterproof coating film is applied on the surface of the hair of the hair portion where said waterproof coating film is formed in such a manner that the surface of each hair is coated with a foundation agent for hair in cylindrical or sheath form in said step of fixing and stabilizing a foundation agent for hair on hair, hair is rubbed together and/or loosened using a comb or a brush so that the hair volume increasing agent is applied to the hair, and the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a foundation agent and a hair volume increasing agent in cylindrical or sheath form, and thus, the hair volume increasing agent is fixed and stabilized on the hair, said hair volume increasing agent in liquid form is applied on the surface of the hair on the hair portion where said hair volume increasing coating film is formed in order to further enhance the effects of increasing hair volume, the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a hair volume increasing agent in cylindrical or sheath form; and the step of fixing and stabilizing a film coating agent on hair, wherein a film coating agent for forming a waterproof coating film having appropriate affinity and adhesiveness with said hair volume increasing coating film is applied to the surface of the hair which has been increased by a desired volume in such a manner that the surface of each hair is coated with a hair volume increasing agent together with a foundation agent in cylindrical or sheath form in the step where said hair volume increasing coating film forming operation is repeated and the hair is loosened with a comb or a brush until the strands of hair to which the film coating agent has been applied are separated from each other and the film coating agent solidifies, making combing smooth, and thereby, a film coating is formed on the surface of each hair which is coated with a film coating agent in cylindrical or sheath form.
(16) A hair volume increasing agent set used for a method for increasing hair volume, comprising: a foundation agent for hair in liquid form made of a lower alcohol solution containing 3 wt % to 35 wt % of a resin component for forming a coating film which is compatible with hair, has flexibility, has little malleability and is highly waterproof; a hair volume increasing agent in liquid form made of a lower alcohol solution containing 95 wt % to 5 wt % of a resin composition for forming a coating film which has appropriate affinity and adhesiveness with a coating film formed on hair of said foundation agent for hair, increases the volume of hair to a great degree, has flexibility, has little malleability and is highly waterproof; and a film coating agent made of a resin component for forming a coating film which has appropriate affinity and adhesiveness with a hair volume increasing coating film that is formed on hair of said hair volume increasing agent, preserves the effects of increasing hair volume and is highly waterproof.

In the present invention, "hair in a portion where it is desired for hair to increase in thickness" also includes hair that is not thin in addition to hair that is thin. Even in the case where hair is not thin, for example, the invention can be applied to those whose hair is thin by nature in comparison with other people and who desire to make their hair thick, as well as those whose hair has a normal thickness and who desire to make their hair thicker.

What is important in the present invention is that the outermost coating film on the hair that has become thick with a hair volume increasing coating film is formed on the hair in cylindrical or sheath form. In the invention according to (1), a waterproof hair volume increasing agent is selected for use as the hair volume increasing agent, and therefore, in the case where a hair volume increasing coating film is formed in cylindrical or sheath form, the length of the cylinder (length of hair) is considerably great in comparison with the diameter of the cylinder (diameter of hair), and therefore, this hair volume increasing coating film never comes off from the hair, and thus, the waterproof properties of the hair volume increasing agent work, and it is not necessary to specially protect the hair volume increasing coating film using a film coating agent. In the invention according to (2), the hair volume increasing agent is not limited to being waterproof as the hair volume increasing agent according to (1). Accordingly, in the case where a non-waterproof hair volume increasing agent is used, the hair volume increasing coating film is not waterproof, though hair volume is increased by the hair volume increasing coating film, and it is troublesome to get wet in the rain, and the user cannot take a bath or swim in a pool. Therefore, it becomes necessary to provide a waterproof coating film in cylindrical or sheath form on this hair volume increasing film. At this time, the outermost coating film is formed in cylindrical or sheath form, and therefore, it is not necessary for the hair volume increasing coating film to be in cylindrical or sheath form. Needless to say, a hair volume increasing coating film finished in cylindrical or sheath form becomes a stronger coating film. In the invention according to (3), an additional waterproof coating film in cylindrical or sheath form is formed on top of the waterproof hair volume increasing coating film, further enhancing the waterproof properties. At this time, a waterproof hair volume increasing coating film finished in cylindrical or sheath form becomes a stronger coating film for the same reasons as with the invention according to (2). In the inventions according to (2) and (3), it takes more time to finish the hair volume increasing coating film in cylindrical or sheath form because meticulous work is accordingly required. The outermost waterproof coating film is formed in cylindrical or sheath form, and thus, whether or not the hair volume increasing coating film is also formed in cylindrical or sheath form is determined taking into consideration points including the cost for implementation of the method for increasing hair volume, because this is reflected in the decision.

What is important in the present invention is that the agent required for increasing hair volume which is fixed and stabilized on the surface of each hair is a coating body in cylindrical or sheath form. A coating film in cylindrical form is made of a hair volume increasing agent which coats the surroundings of the torso portion of the hair, excluding the end portion, and a coating film in sheath form is made of a hair volume increasing agent which coats the surroundings of the torso portion, including the end portion of the hair. Sheath form means a state of a cylindrical form with one end closed, and it can be said that a sheath form is made of a portion in cylindrical form and a portion enclosing the tip of this portion in cylindrical form. The portion enclosing the tip of a portion in sheath form may or may not exist, and accordingly, the portion in cylindrical form is important. In the case of short, thin hair, it is difficult to provide a cylindrical form, and a sheath form is naturally provided.

The viscosity of the components of the present invention varies a great deal depending on the properties of the resins and the bulking agent and/or the concentration and state of mixture of these, added precipitation preventing agent, the type of thickening agent and the like. Accordingly, liquid form in the present invention refers to a substance in liquid form in a state of low viscosity or middle viscosity, irrespectively of whether it is sol or gel, and in such a state that it is fluid and can be taken out from a container.

In the present invention, "foundation agent" refers to a component for forming a resin coating film for raising hair. "Hair volume increasing agent" refers to a component for increasing hair volume. In addition, "film coating agent" refers to a component for forming a resin coating film for protecting a hair volume increasing coating film that is formed of a hair volume increasing agent.

In the present invention, "compatible with hair" refers to a state of excellent affinity and adhesiveness with hair, and in the present invention, refers to a state where an agent made of a resin in liquid form for forming a coating film spreads along and makes contact with the surface of hair without meeting with resistance, for example being expelled by the hair, when at least several drops of the agent are applied to bundled hair. "Having flexibility" means that slackening is possible, and in the present invention, refers to a state where the resin coating film bends without peeling off from the surface of hair in such a manner that no peeling or cracking occurs in the resin coating film on the surface of hair, even when an agent made of a resin in liquid form for forming a coating film is applied to at least five hairs which are aligned so as not to overlap with each other and are bundled, and both ends of these hairs are held by hands and the hairs are bent several times to several tens of times after being completely dried. "Having little malleability" means that wear or destruction results due to external force, such as an impact, vibration, pressure and the like, and is used when such a phenomenon occurs with a slight external force, and in the present invention, refers to a state where strands of hair are separated from each other when the resin that has solidified between hairs easily cracks, is shaved, worn or the like when at least about twenty hairs are bundled and an agent made of a resin in liquid form for forming a coating film is applied to these hairs, and then, the two ends of the bundle of hairs are held by hands after being completely dried and rubbed and stroked several times to several tens of times or stroked with such a movement as to bend the bundle of hair. It becomes possible due to the low malleability to separate hairs from each other in a state where each hair is coated with a film by rubbing and loosening the hair, even in the case where the hairs are connected to each other in such a state as to overlap. In the present invention, "waterproof properties" refers to a state where no change, such as peeling of resin, occurs on the surface of approximately twenty hairs which are bundled and to which an agent made of a resin in liquid form for forming a coating film has been applied for at least three hours when the hairs are soaked in water at 25 °C after the hairs are soaked with a sufficient amount of agent so that the agent is applied thereto, and left and completely dried using a drier.

The "method for increasing hair volume" according to the present invention is further described in the following. In the case where a waterproof hair volume increasing agent is selected as the hair volume increasing agent and this hair volume increasing agent is finished on hair in cylindrical or sheath form, the hair volume increasing coating film is waterproof and the hair volume increasing coating film is in cylindrical or sheath form, and therefore, does not come off easily from the hair. Even in the case where a water soluble hair volume increasing agent is selected, a waterproof coating film in cylindrical or sheath form is provided on top of the hair volume increasing coating film that is formed of this hair volume increasing agent, and therefore, no problem arises in terms of the waterproof properties and strength. In the case where a waterproof hair volume increasing agent is selected as the hair volume increasing agent and an additional waterproof coating film is provided on top of the waterproof hair volume increasing coating film, waterproof properties are further enhanced and the strength of the waterproof coating film also increases. In the case where non-neutralizing resins are used for both the waterproof hair volume increasing agent and the waterproof film coating agent, the waterproof coating film and the waterproof hair volume increasing coating film are both neutralized with an alkaline neutralizer in the case where the user desires the removal thereof for his/her own convenience, and thus, can be removed by changing them so that they are water soluble. Removable with an alkaline neutralizer means that the non-neutralizing resin coating film can be removed as a water soluble film with alkaline sweat (which is the same as an alkaline neutralizer) in the case where, for example, an athlete expires a large amount of alkaline sweat, referred to as bad sweat, as a result of hard training. As for the means for solving this problem, acid-proof, alkaline-proof and waterproof resin or a photo-setting resin which does not become water soluble even when neutralized with an alkaline neutralizer is used for the outermost coating film on the hair volume increasing film. Now, the photo-setting resin may have acid-proof and alkaline-proof properties or not.

Nitrocellulose or latexes which become a waterproof coating film after being cured from among multipurpose latexes, such as NR and other general diene based synthetic polymers, as well as polymer latexes, including MBR, BR, VP, NIR, SIR, IR, FKM, EPM and CSM can be used as an acid-proof, alkaline-proof and waterproof film coating agent which does not become water soluble, even when neutralized with an alkaline neutralizer that can be used in the present invention.

The photo-setting resin that can be used in the present invention is not particularly limited, and resins from which a coating film can be fabricated using a photo-setting technology with visible and/or ultraviolet rays may be used. It is preferable for the resin component to be in liquid form before being cured, meaning that it is easy to handle and has little stimulation, irrespectively of whether it is oil soluble or water soluble, under conditions such that the resin is safe when used for the human body, and has no smell and little discomfort. Though the method for polymerization is not particularly limited, the resulting appearance may vary depending on the intensity of light, the viscosity of the medium and the concentration of the initiator, and thus, it is preferable, in terms of the method, to use optical radical polymerization such as that currently used for the human body in the field of dentistry. The resin component is not particularly limited. A methacrylate based resin, for example, can be used. As for the methacrylate based resin, though a monomer having one functional group and a relatively large molecular weight is preferable, resins having two functional groups or multiple functional groups do not particularly cause any problems. Bis-GMA, which is an addition compound of bisphenol-A diglycidyl ether and methacrylic acid, triethylene glycol dimethacrylate (TEGDMA) and urethane dimethacrylate (uDMA), for example, are mainly used as the methacrylate based resins. These are derivatives from methyl methacrylate (MMA) and TEGDMA, for example, has a molecular structure of two functional groups with two double bonds where two MMA's are bonded, and can form a cross linking structure which is hard and insoluble in an organic solvent through reaction between the two double bonds. Though a monomer having one functional group and a relatively large molecular weight is preferable for preventing the coating for hair from becoming hard, resins having two functional groups and multiple functional groups do not particularly cause any problems.

As for the monomer, oligomer and polymer, acrylic acid, methacrylic acid, ester acrylate based resins, ester methacrylate based resins, polyacrylic acid based resins, novolac type phenol epoxy resins, novolac cresol epoxy resins, polyimide based resins, polyvinyl alcohol based resins, multifunctional polyester acrylate based resins, multifunctional polyol acrylate based resins and multifunctional polyurethane acrylate based resins, such as TEGDMA, BuDMA, NPGDMA, HDDMA, UDMA, Bis-DMA, Bis-MPEE4E and TCDDMA, as well as mixed resins of these, can be used. As for the polymerization initiator, 1-hydroxycyclohexyl phenyl ketone, 2-hydroxy-2-methyl-1-phenyl-propane-1-on, 2-methyl-1-(4-(methyl thio)phenyl)-2-morpholinoprothrin-1, 2-benzyl-2-dimethyl amino-1-(4-morpholinophenyl)-butanone-1 and the like can be used. As for the dispersing agent, PEO alkyl phenyl ether based resins, POG diester based resins, sorbitan fatty acid ester based resins, fatty acid modified polyester based resins, tertiary amine modified polyurethane based resins and the like can be used. In addition, as for the sensitizing agent, adhesion enhancing agent, reducing agent and the like, α-diketones, silane coupling agents, camphor quinone, 2-(N, N-dimethyl amino) ethyl methacrylate, alkyl barbituric acid and the like can be used, and it is also possible to mix in a pigment or the like in order to increase the color purity, the concentration, the weather resistance and spectral properties. As for the light source for irradiation, a halogen lamp or a laser light source can be used, and as for the medium for light transmission, an optical fiber or the like can be used.

It is possible in implementing the present invention to take various measures on use a water soluble photo polymerization initiator in consideration of environmental issues, and on the multipurpose laser light source to limit visible region or shorten the time for irradiation, in order to prevent damage by ultraviolet rays through localized irradiation from becoming a problem.

Here, the effects of the present invention are briefly summarized as follows.
(1) A hair volume increasing coating film in cylindrical or sheath form is formed on each hair, and therefore, hair volume can be increased in a quite natural state without being different from the user's original hair and in a short period of time, such as 20 minutes to 30 minutes. (2) The volume of waterproof hair can be increased in such a manner that no problem arises when hit by rain, the user swims in a pool, takes a bath or washes the hair. The hair volume increasing coating film protects the surface of the user' s original hair, making combing and brushing smooth, and thus, hair can be prevented from being ripped during brushing, the growth of hair is aided and the hairdo can have a beautiful finish. (3) The hair volume increasing coating film does not fall off from the user's original hair, and is provided with endurance. (4) It is inexpensive in comparison with facial hair or implanted hair. (5) The method for increasing hair volume can be easily implemented and used with ease. It can be said that the method for increasing hair volume has surprisingly excellent effects as described above.

### Brief Description of the Drawings

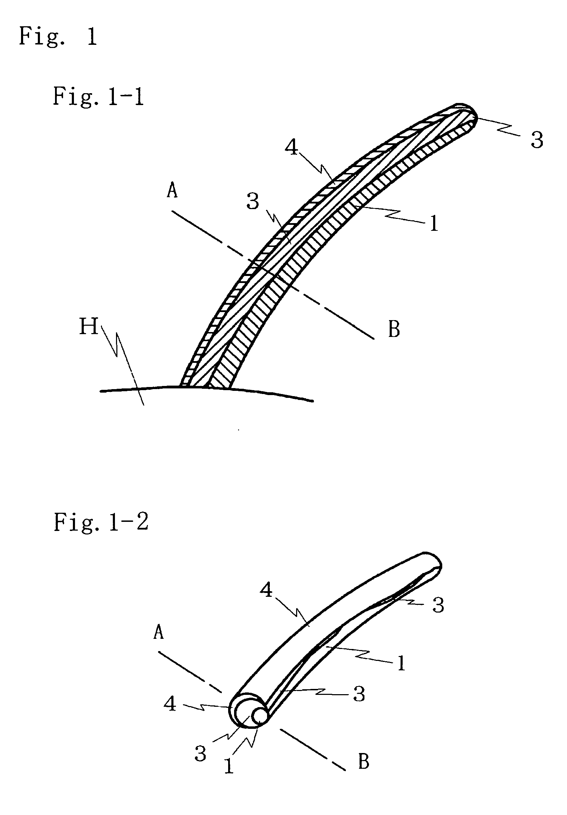

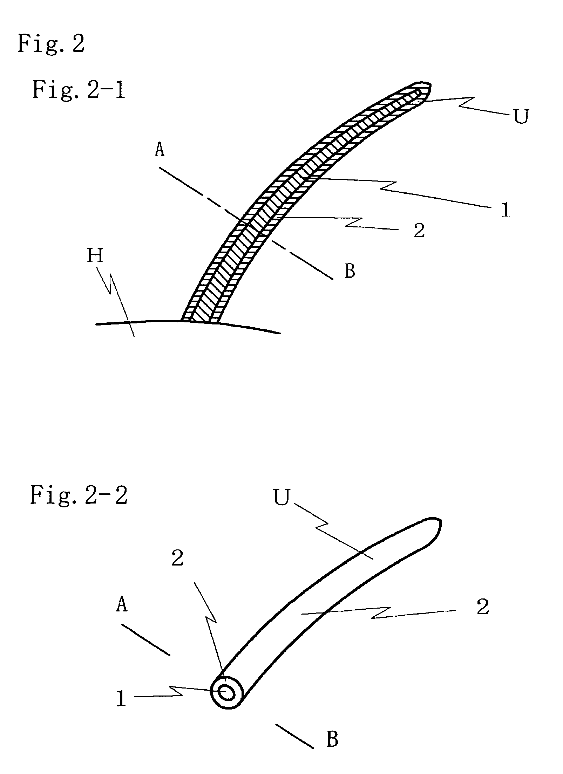

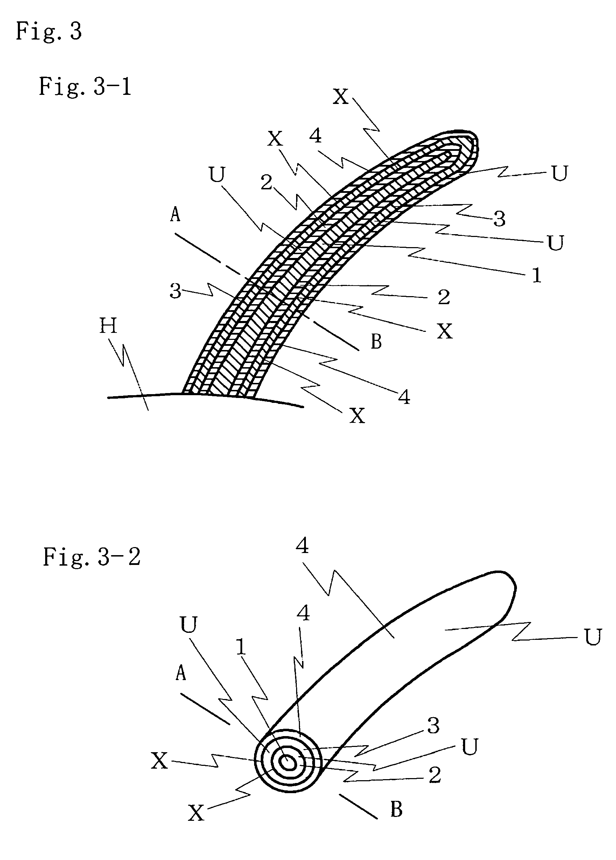

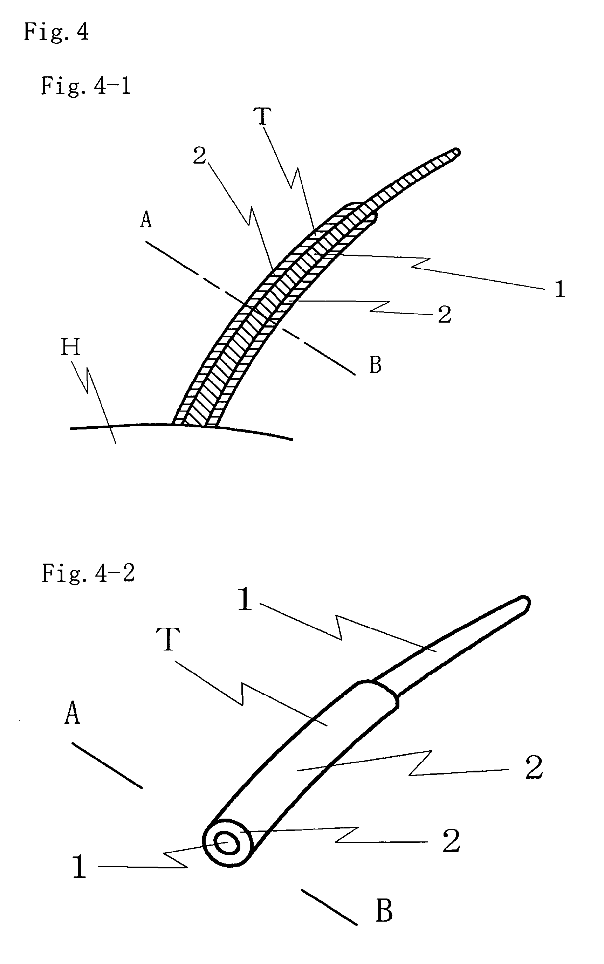

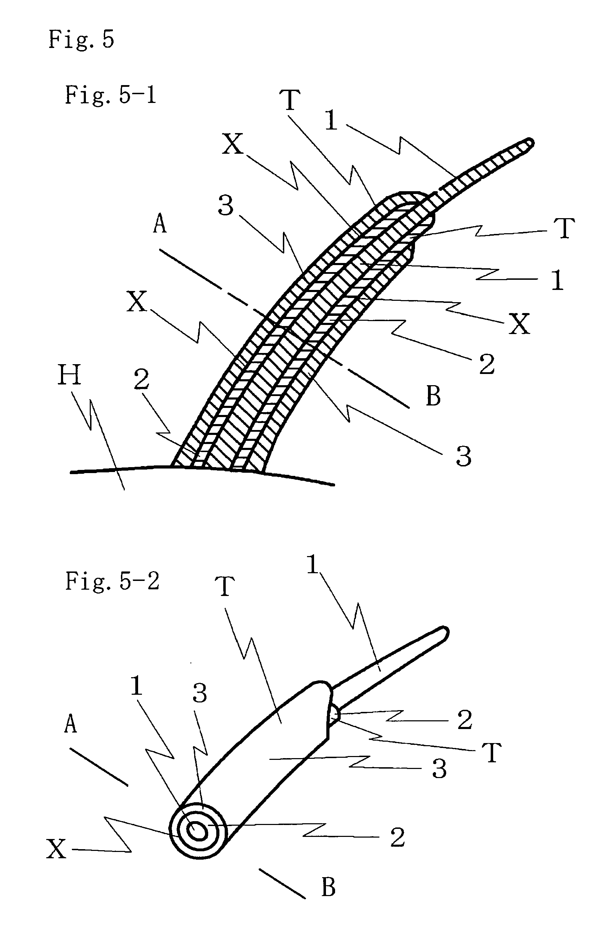

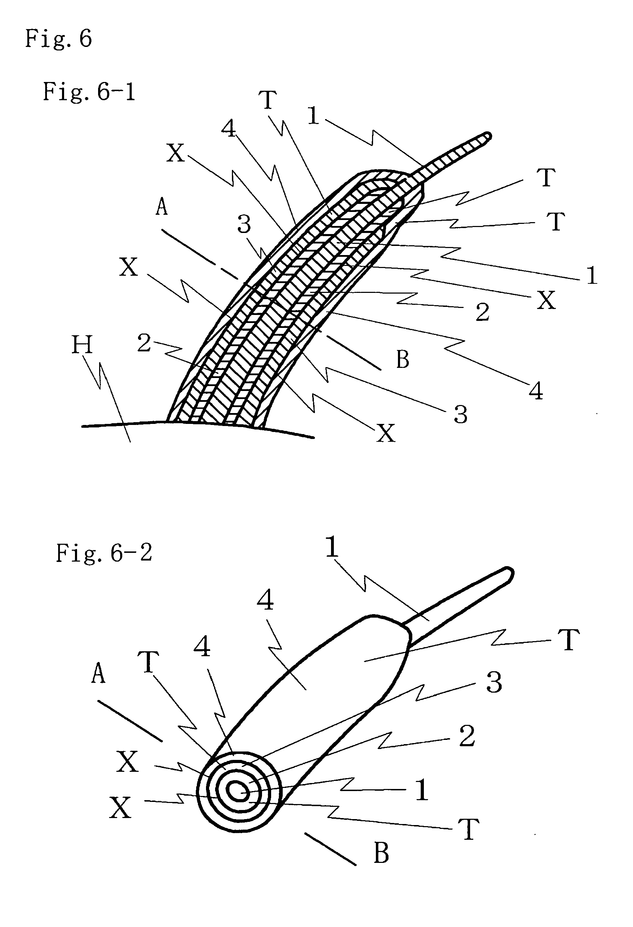

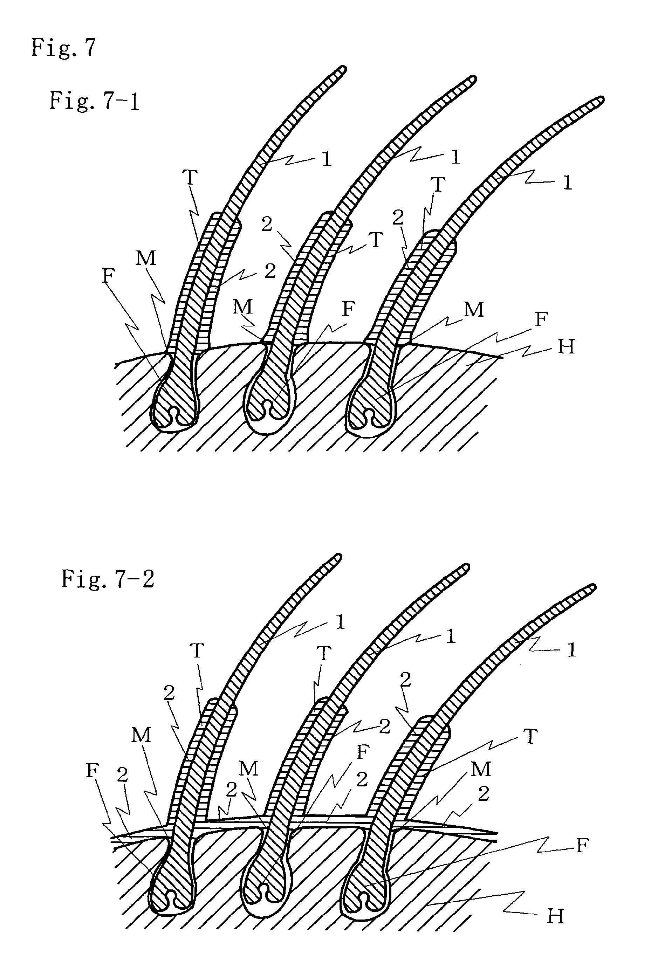

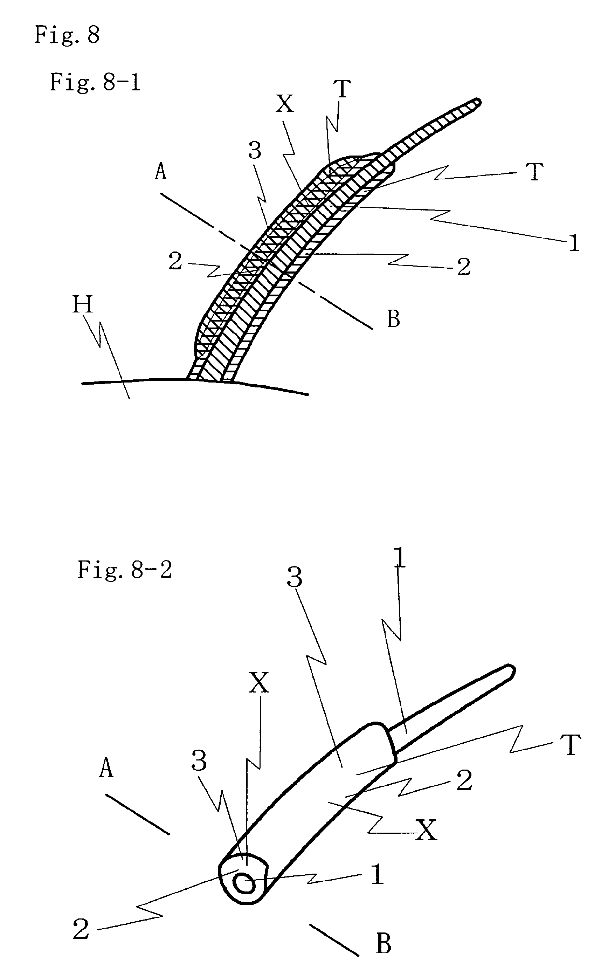

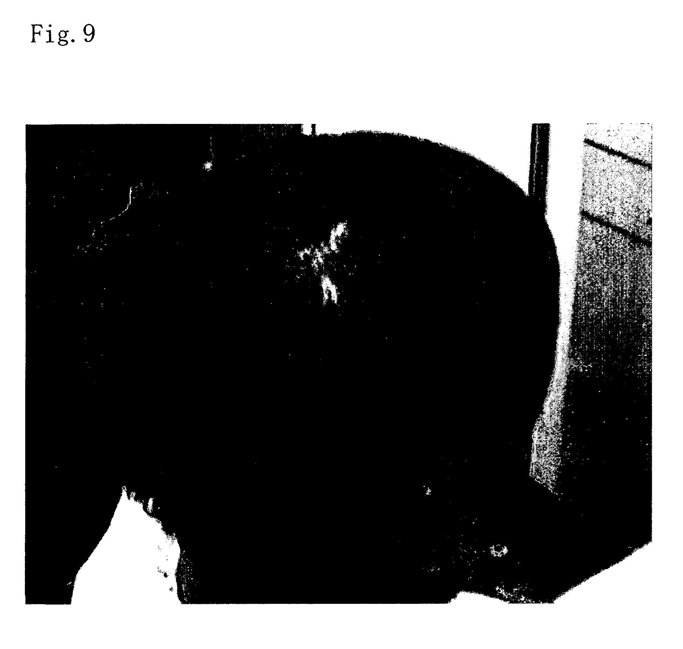

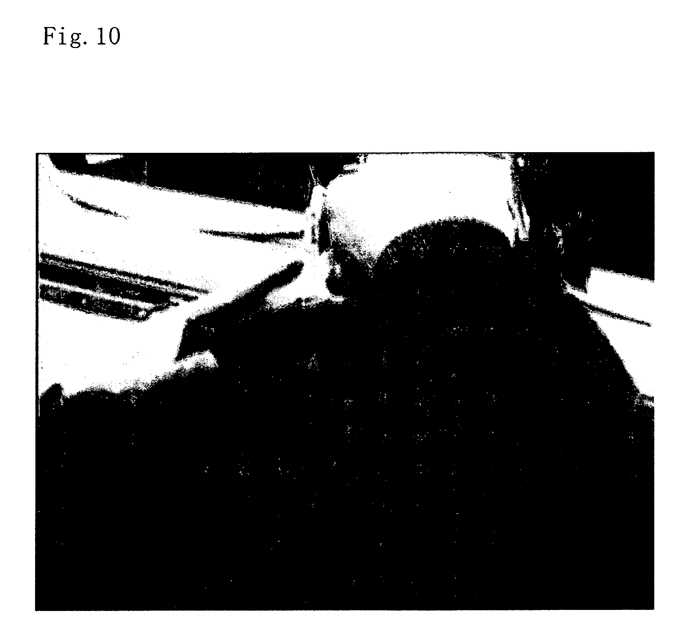

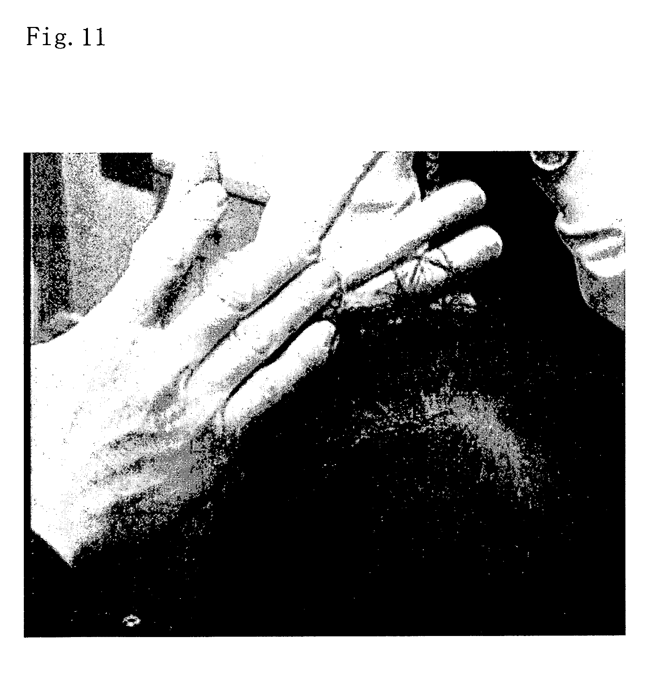

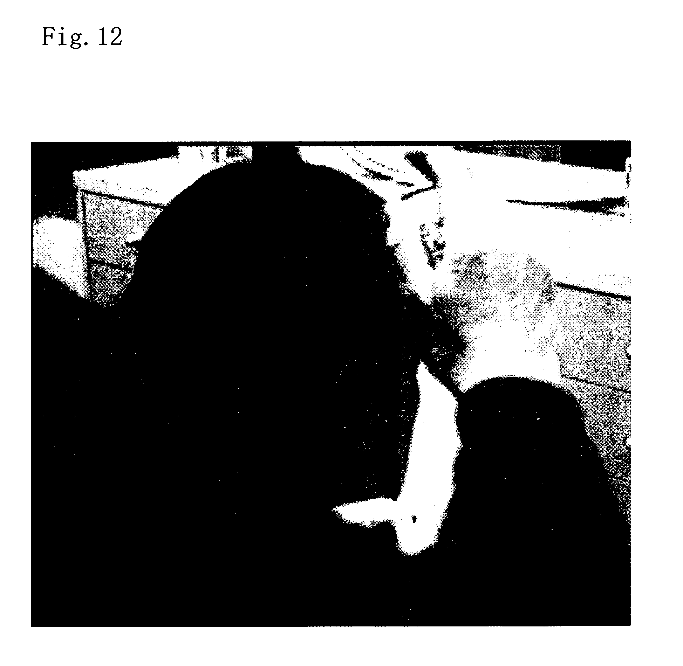

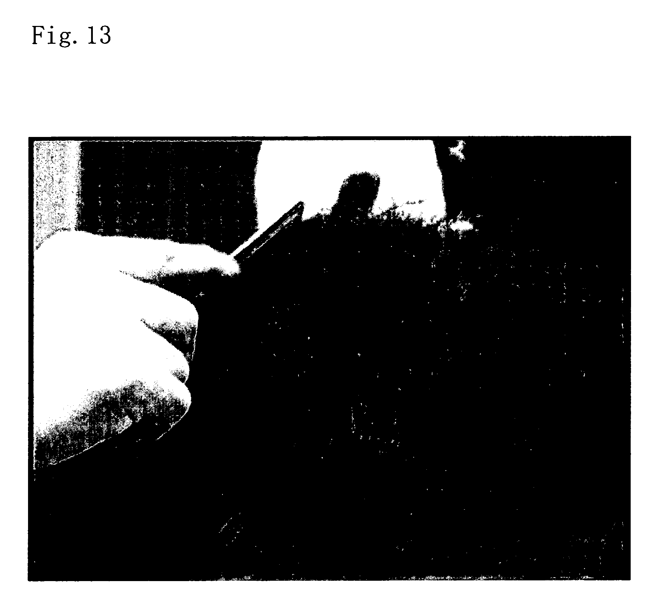

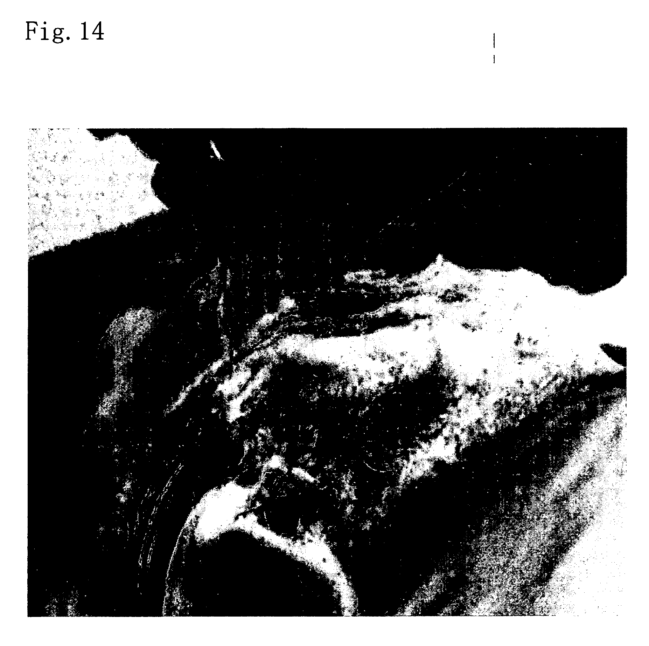

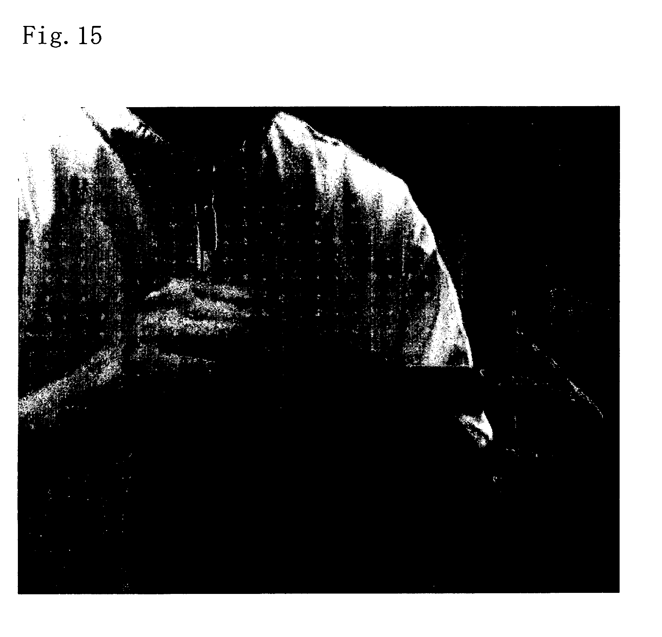

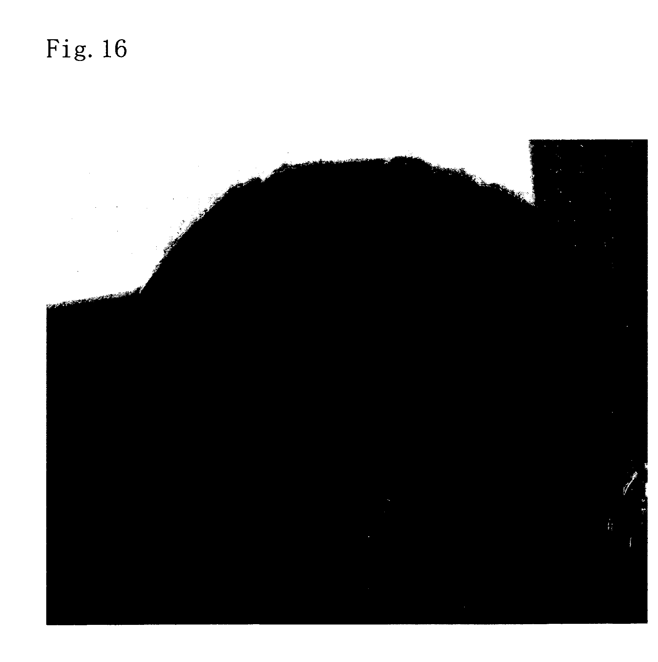

Figs. 1-1 and 1-2 are conceptual diagrams for illustrating a method for and the effects of increasing hair volume according to the prior art; Fig. 1-1 is a longitudinal cross sectional diagram for illustrating a method for and the effects of increasing hair volume according to the prior art showing a hair portion, and Fig. 1-2 is a conceptual diagram for illustrating a method for and the effects of increasing hair volume according to the prior art showing the appearance of a hair; Figs. 2-1 and 2-2 are conceptual diagrams for illustrating a method for and the effects of increasing hair volume according to the present invention; Fig. 2-1 is a longitudinal cross sectional diagram for illustrating a method for and the effects of increasing hair volume according to the present invention showing a hair portion, and Fig. 2-2 is a conceptual diagram for illustrating a method for and the effects of increasing hair volume according to the present invention showing the appearance of a hair; Figs. 3-1 and 3-2 are conceptual diagrams for illustrating a method for and the effects of increasing hair volume according to the present invention; Fig. 3-1 is a longitudinal cross sectional diagram for illustrating a method for and the effects of increasing hair volume according to the present invention showing a hair portion, and Fig. 3-2 is a conceptual diagram for illustrating a method for and the effects of increasing hair volume according to the present invention showing the appearance of a hair; Figs. 4-1 and 4-2 are conceptual diagrams for illustrating a method for and the effects of increasing hair volume according to the present invention; Fig. 4-1 is a longitudinal cross sectional diagram for illustrating a method for and the effects of increasing hair volume according to the present invention showing a hair portion, and Fig. 4-2 is a conceptual diagram for illustrating a method for and the effects of increasing hair volume according to the present invention showing the appearance of a hair; Figs. 5-1 and 5-2 are conceptual diagrams for illustrating a method for and the effects of increasing hair volume according to the present invention; Fig. 5-1 is a longitudinal cross sectional diagram for illustrating a method for and the effects of increasing hair volume according to the present invention showing a hair portion, and Fig. 5-2 is a conceptual diagram for illustrating a method for and the effects of increasing hair volume according to the present invention showing the appearance of a hair; Figs. 6-1 and 6-2 are conceptual diagrams for illustrating a method for and the effects of increasing hair volume according to the present invention; Fig. 6-1 is a longitudinal cross sectional diagram for illustrating a method for and the effects of increasing hair volume according to the present invention showing a hair portion, and Fig. 6-2 is a conceptual diagram for illustrating a method for and the effects of increasing hair volume according to the present invention showing the appearance of a hair; Figs. 7-1 and 7-2 are conceptual diagrams for illustrating a method for and the effects of increasing hair volume according to the present invention; Fig. 7-1 is a longitudinal cross section for illustrating a method for and the effects of increasing hair volume according to the present invention showing scalp and a hair portion, and Fig. 7-2 is a longitudinal cross section for illustrating a method for and the effects of increasing hair volume according to the present invention showing scalp and a hair portion; Figs. 8-1 and 8-2 are conceptual diagrams for illustrating a method for and the effects of increasing hair volume according to the present invention; Fig. 8-1 is a longitudinal cross sectional diagram for illustrating a method for and the effects of increasing hair volume according to the present invention showing a hair portion, and Fig. 8-2 is a conceptual diagram for illustrating a method for and the effects of increasing hair volume according to the present invention showing the appearance of a hair; Fig. 9 is a recording photograph 1 instead of a diagram, showing Example 2 of the present invention; Fig. 10 is a recording photograph 2 instead of a diagram, showing Example 2 of the present invention; Fig. 11 is a recording photograph 3 instead of a diagram, showing Example 2 of the present invention; Fig. 12 is a recording photograph 4 instead of a diagram, showing Example 2 of the present invention; Fig. 13 is a recording photograph 5 instead of a diagram, showing Example 2 of the present invention; Fig. 14 is a recording photograph 6 instead of a diagram, showing Example 2 of the present invention; Fig. 15 is a recording photograph 7 instead of a diagram, showing Example 2 of the present invention; and Fig. 16 is a recording photograph 8 instead of a diagram, showing Example 2 of the present invention.

### Explanation of Symbols

- 1: hair
- 2: foundation agent
- 3: hair volume increasing agent
- 4: film coating agent
- A-B: showing point at which cross section taken
- F: hair root
- H: scalp
- M: vicinity of hair root
- T: coating film portion in cylindrical form
- U: coating film portion in sheath form
- X: region where agents bond

### Best Mode for Carrying Out the Invention

In the following, concrete embodiments to which the present invention is applied are described in reference to the drawings.

Figs. 1-1 and 1-2 are conceptual diagrams for illustrating a method for and the effects of increasing hair volume according to the prior art, and Figs. 2-1 to 8-2 are conceptual diagrams for illustrating a method for and the effects of increasing hair volume according to the present invention. Here, in order to make description of the present invention easier, the component for forming a resin coating film for raising hairs 1 is referred to as a foundation agent, the component for forming a coating film in order to increase hair volume is referred to as a hair volume increasing agent, and the component for forming a resin coating film in order to protect the hair volume increasing agent is referred to as a film coating agent in the following description. Here, Figs. 1-1, 2-1, 3-1, 4-1, 5-1, 6-1 and 8-1 are longitudinal cross sectional diagrams showing hair portions on the scalp in different states, and Figs. 7-1 and 7-2 are longitudinal cross sectional diagrams showing the scalp and hair portions. Figs. 1-2, 2-2, 3-2, 4-2, 5-2, 6-2 and 8-2 show cross sections when cut at points a-b in the respective states of Figs. 1-1, 2-1, 3-1, 4-1, 5-1, 6-1 and 8-1, and the appearance of hairs in the portion above a-b. In the following, 1 indicates a hair, 2 indicates a foundation agent, 3 indicates a hair volume increasing agent, 4 indicates a film coating agent, F indicates the hair root portion, H indicates the scalp, M indicates the vicinity of the hair root, T indicates a coating film portion in cylindrical form, U indicates a coating film portion in sheath form, and X indicates a region where agents bond in the figures.

Figs. 1-1 and 1-2 are conceptual diagrams showing the state of a hair in the case where the hair is treated in accordance with "a method for increasing hair volume according to the prior art" using a conventional "hair volume increasing agent 3 of a type where a short fiber material or a powder material are sprayed and applied." All of the above described conventional "temporary hair volume increasing methods" are methods "for making hair look fuller by thoroughly and uniformly applying a hair volume increasing component on the surface of hair," and the effects of increasing hair volume are lost or lowered when the hair is combed after the hair volume increasing treatment, and therefore, the hair volume increasing agent (hair volume increasing component) is usually applied to the hair after setting.

According to the method for treatment, a hair volume increasing agent 3, which is a hair volume increasing component, is applied to hair 1 after setting, and after a while, in order to prevent the hair volume increasing agent 3 (powder or the like) from falling off from the hair 1, a film coating agent 4 (hairspray) is lightly applied on top of the applied hair volume increasing agent 3, and film coating treatment is carried out. Accordingly, Figs. 1-1 and 1-2 show the state of hair 1 after hair volume increasing agent 3 is applied, and then the film coating agent 4 is applied while paying attention so that the hair volume increasing agent 3 does not come off from the hair 1 without combing the hair and the hair is dried as it is.

Figs. 2-1 to 8-2 are conceptual diagrams for illustrating a case of treatment using a method according to the present invention. Figs. 2-1 to 3-2 show the state of a hair in the case where "short hair is treated and film coating treatment is carried out" according to the present invention, and Figs. 4-1 to 8-2 show the state of a hair in the case where long hair is partially treated and film coating treatment is carried out. Here, in order to make description of the contents of the present invention easier, in the description henceforth, description is limited to the "state of hair in the case where short hair is treated and film coating treatment is carried out" in reference to Figs. 2-1 to 3-2. That is to say, Figs. 2-1 to 3-2 show a "case where short hair is treated in accordance with the present invention," and thus, a foundation agent 2, a hair volume increasing agent 3 and a film coating agent 4 sufficiently spread over the entirety of the surface of the hair 1, and therefore, the figures show a coating film in sheath form (in other words, a long cylindrical form with one end closed) rather than in cylindrical form on hair 1. Figs. 2-1 and 2-2 are conceptual diagrams showing the state of hair after the surface is treated with a foundation agent according to the present invention, and Figs. 3-1 and 3-2 are conceptual diagrams showing the state after the hair of Figs. 2-1 and 2-2 is further treated with a hair volume increasing agent and a film coating agent.

Next, in order to describe comparison of the effects, the method for treatment is outlined, starting from Figs. 2-1 and 2-2 to Figs. 3-1 and 3-2.

A method for treatment for implementing the present invention is outlined in the following. A sufficient amount of a foundation agent is applied to the hair on the portion of the head where hair grows and it is desired for hair to increase in thickness, the hair is loosened with the hands, a comb, a brush or the like until the strands of hair to which the foundation agent has been applied are separated from each other and the foundation agent solidifies, so that the foundation agent spreads from the vicinity of the hair root to the tip of the hair and dries on each hair, and after that, a hair volume increasing agent is applied and the hair is loosened with the hands, a comb, a brush or the like until the hair volume increasing agent solidifies on the surface of each hair, and thus, the hair volume increasing agent spreads and dries on the surface of each hair. After that, a film coating agent is further applied and the hair is loosened with the hands, a comb, a brush or the like until the agent solidifies, and thus, the film coating agent spreads and dries on the entirety of the surface of each hair, of which the state is shown. As for Figs. 2-1 to 3-2, Figs. 2-1 and 3-1 are longitudinal cross sectional diagrams showing the hair portion on the scalp in different states, and Figs. 2-2 and 3-2 show cross sectional surfaces where the hair in the respective states of Figs. 2-1 and 3-1 is cut at the point a-b, and the appearance of the hair above the point a-b.

According to the conventional method for increasing hair volume, a hair volume increasing agent 3 is applied on the surface of the hair 1 after setting, as shown in Figs. 1-1 and 1-2, and then a film coating agent 4 is applied, and thus treated without being loosened with a comb, and thus, the hair volume increasing agent 3 and the film coating agent 4 are applied to hair 1 in such a state that hair 1 is coated with top skins of the hair volume increasing agent 3 and a film coating agent 4, in other words, such a state as if a sliced omelet were placed on top of a ball of rice and a sheet of dried seaweed were placed on top, as compared to the rice and ingredients for hand-shaped sushi, and therefore, the strength of adhesion between the surface of hair 1 and the hair volume increasing agent 3 is only that of the adhesive resin contained in the hair volume increasing agent 3, and in addition, even in the case where the strength of adhesion of the hairspray, which is the film coating agent 4, is stronger than the strength of adhesion of the hair volume increasing agent 3, the hair volume increasing agent 3 cannot be sufficiently prevented from coming off from hair 1, unless the hairspray coats the hair volume increasing agent 3 in such a form as to completely cover the latter without missing any portions.

In the case where the present invention is implemented, so that hair 1 is rubbed together and hair 1 is loosened with a comb or a brush after a foundation agent 2, a hair volume increasing agent 3 and a film coating agent 4 are respectively applied, so that the surface of each hair 1 is coated with films in such a form that the respective agents sufficiently wrap around hair 1, that is to say, in cylindrical form or sheath form. In the case where the present invention is implemented, as shown in Figs. 2-1 to 3-2, the surroundings of hair 1, the foundation agent 2 and the hair volume increasing agent 3 are coated with the foundation agent 2, the hair volume increasing agent 3 and the film coating agent 4 in such a state that each agent forms a sheath as that of a sword (in other words, a long cylindrical form with one end closed, which remains the same in the following), and thus, the foundation agent 2 covers hair 1, the hair volume increasing agent 3 covers hair 1 and the foundation agent 2, and the film coating agent 4 covers hair 1, the foundation agent 2 and the hair volume increasing agent 3 in sheath form (coating portion U in sheath form), and thus, the foundation agent 2, the hair volume increasing agent 3 and the film coating agent 4 do not come off, for the following reasons, irrespectively of the strength of adhesion between the respective agents (between hair 1 and the foundation agent, between the foundation agent and the hair volume increasing agent, between the hair volume increasing agent and the film coating agent and the like), even though the strength of adhesion is weak. That is to say, the respective bodies in sheath form of the foundation agent 2, the hair volume increasing agent 3 and the film coating agent 4 have a considerably great length of the sheath (cylinder portion) (length of the portion coated with the agent applied to hair 1) relative to the diameter of the sheath (cylinder) (almost the same as the diameter of hair 1), and therefore, the resistance against drawing off of the respective sheaths (long cylindrical portion) from hair 1 (referred to as effects of resistance against drawing off, which remains the same in the following) is great, and thus, the sheaths never come off from hair 1.

As is clear from the above reasons, though only a temporary hair volume increasing portion with effects that last for only a short time can be made according to the conventional method, even when a waterproof resin is used for the hair volume increasing agent or the film coating agent, according to the present invention, the foundation agent, the hair volume increasing agent and the film coating agent form a highly waterproof coating film, and the foundation agent, the hair volume increasing agent and the film coating agent separately or collectively form a coating film in sheath (cylindrical) form and therefore, it is evident that a hair volume increasing portion having semi-permanent effects of increasing hair volume can be easily made.

The conventional method shown in Fig. 1 and hair volume increasing treatment according to the method of the present invention shown in Figs. 2-1 to 3-2 were actually carried out, and the hair was washed for approximately five minutes with hot water, and then, most of the hair volume increasing component was washed away in the former, that is to say, in the conventional method, and the effects of increasing hair volume were almost completely lost, while the effects of increasing hair volume were not visibly lost in the latter, that is to say, in the method of the present invention, though a slight amount of the hair volume increasing treatment agent was washed away with the hot water (not even a slight amount was washed away during subsequent washing with hot water, except during washing immediately after the hair volume increasing treatment).

Here, the point that is more important in the present invention is described. Since a case where "the present invention is implemented for short hair 1" is exemplified and illustrated in order to make description of the contents of the present invention easier, the figures 2-1 to 3-2 show that the foundation agent 2, the hair volume increasing agent 3 and the film coating agent 4 are coating films in complete sheath form. However, it was found that treatment of all of the hair on the head portion where hair grows and it is desired for hair to increase in the thickness in complete sheath form when the present invention is implemented for actual hair on a head consumes a large amount of agents and takes a long time for treatment, and as a result of diligent research, it was found, in terms of the above described "effects of resistance against drawing off," the foundation agent 2, the hair volume increasing agent 3 and the film coating agent 4 never come off from hair 1 only in the case where a coating film in cylindrical form is formed on the hair, so that this coating film in cylindrical form has a sufficiently great length of the cylinder (length of the coating film in cylindrical form on hair 1) relative to the cylinder diameter (diameter of hair 1), even though the coating films of the foundation agent, the hair volume increasing agent and the film coating agent are not finished in sheath form.

Figs. 4-1 to 6-2 show examples of the present invention where hair was finished in this manner. Figs. 4-1 and 4-2 are conceptual diagrams showing the state of hair in the case where a foundation agent is partially applied to long hair and film coating treatment is carried out on the hair partially according to the present invention, Figs. 5-1 and 5-2 are conceptual diagrams showing the state of hair where a hair volume increasing agent is further applied to the hair of Figs. 4-1 and 4-2, and Figs. 6-1 and 6-2 are conceptual diagrams showing the state of hair where the hair of Figs. 5-1 and 5-2 is further treated with a film coating agent. From among Figs. 4-1 to 6-2, Figs. 4-1, 5-1 and 6-1 are longitudinal cross sectional diagrams showing a portion of hair on the scalp in different states, and Figs. 4-2, 5-2 and 6-2 show cross sections of hair when cut at the point a-b in the respective states of Figs. 4-1, 5-1 and 6-1, as well as the appearance of hair above the point a-b. In addition, 1 indicates hair, 2 indicates a foundation agent, 3 indicates a hair volume increasing agent, 4 indicates a film coating agent and H indicates the scalp.

Here, the necessity for and the meaning of foundation agent according to the present invention are described.

It is preferable to carry out foundation treatment on hair and make the state on the surface of the hair uniform, in order to gain uniform effects and enhance the effects when hair volume increasing treatment is carried out on hair according to the present invention. Taking this into consideration, the present inventor proposed in Japanese Patent Application 2003-436972 (filed on December 31st, 2003) that "it is appropriate for the component of this foundation material to be compatible with the adhesive resin used in the hair volume increasing agent, and the same or same type of resin as used for the hair volume increasing agent used when implementing the present invention is possible for use as the foundation material when diluted with a solvent (lower alcohol is preferable). That is to say, as the component that can be used as the foundation material, synthetic polymer compounds, including polyvinyl pyrrolidone, polyvinyl alcohol, polyacrylamide, polyacrylates, an acryl resin alkanolamine liquid, a copolymer liquid of N-methacryloyloxyethyl N, N-dimethyl ammonium-α-N-methyl carboxybetaine and alkyl ester methacrylate, a copolymer of methacryloyl ethylene dimethyl betaine, methacryloyl ethyl trimethyl ammonium chloride and 2-hydroxyethyl methacrylate, a copolymer liquid of methacryloyl ethyl dimethyl betaine, methacryloyl ethyl trimethyl ammonium chloride and methoxy polyethylene glycol methacrylate, a copolymer of methoxyethylene and maleic anhydride, natural polymer compounds, including carrageenan, xanthan gum, gelatin, dextrin, natural rubber, ester rubber, modified starch and milk casein, and semisynthetic polymer compounds which are resins that can be used as an adhesive resin for the hair volume increasing agent can be adopted. These resins can be used alone, or two or more can be combined, and in addition, it is also possible to use a copolymer between these or with other substances." In addition, the inventor mentioned that any component can be used, irrespectively of whether it is water soluble or non-water soluble.

Subsequent research, however, revealed that though foundation treatment is naturally effective in "making the effects uniform" and "enhancing the effects of increasing hair volume" when hair volume increasing treatment is carried out according to the present invention, as in the above described case where a "foundation material" is used (Japanese Patent Application 2003-436972), a foundation agent is necessary and important in a completely different sense than the foundation treatment where the above described foundation material is used, that is to say, in order to further enhance the effects of increasing hair volume so as to make the increase in the hair volume conspicuous, and in order to prolong the effects. Unlike in Japanese Patent Application 2003-436972, it does not work as a helping agent before the application of a hair volume increasing agent, but is necessary to raise hair and prolong the effects.

The number of hairs is an important factor in making hair look thick, though thick hair is necessary. Even though it is said that "hair loss at a late middle-age, which appears in the thirties or later, is in many cases not actual loss of hair, neither for men nor for women, but rather, the thickness and the length of hair gradually become smaller, making hair thinner," the number of hairs also considerably reduces, and therefore, a means for compensating for this is necessary. Taking this into consideration, it was found that making hair stand or raising hair is an effective technique for increasing hair volume. Accordingly, a method for raising each hair was examined, and as a result, it was found that an amount of foundation agent that is sufficient to soak hair is applied, and hair is rubbed together and/or hair is loosened using a comb or a brush so that the foundation agent is applied throughout the hair from the vicinity of the hair root to the tip of the hair, and then, hair is loosened with a comb or a brush until the strands of hair to which the foundation agent for hair is applied are separated from each other and the foundation agent solidifies, making combing smooth, and thus, a coating film of the foundation agent which is compatible with hair, has flexibility and is highly waterproof, and with which the surface of each hair is coated from the vicinity of the hair root to the tip of the hair in cylindrical form is formed so that the foundation agent for hair is fixed and stabilized on the hair before being increased in thickness, and treatment in accordance with such a method makes the hair coated with the foundation agent in cylindrical form from the portion on the side of the hair root, that is to say, the vicinity of the hair root, to the tip of the hair, so that the effects of raising hair can be gained. The reason for this is described below.

Returning to the earlier discussion, it was found that the following is important in order to form coating films from the foundation agent 2, the hair volume increasing agent 3 and the film coating agent 4 in cylindrical form on the surface of each of a great number of hairs, so that the above described conditions for gaining "the effects of resistance against drawing off" are met.

That is to say, a sufficient amount of a foundation agent for hair made of a resin in liquid form which forms a coating film that is compatible with hair, has flexibility, has little malleability and is highly waterproof for soaking the hair is applied to the surface of hair on the portion of a head where hair grows, where it is desired for the hair to increase in thickness, hair is rubbed together and/or loosened using a comb or a brush so that the foundation agent for hair is applied on all of the hair, and the hair is loosened with a comb or a brush until strands of hair to which the foundation agent for hair has been applied are separated from each other, the foundation agent solidifies, making combing smooth, and thereby, a coating film that is compatible with hair, has flexibility and is highly waterproof, is formed on the surface of each hair with a foundation agent for hair in cylindrical form from the vicinity of the hair root to the tip of the hair, and thus, a foundation agent for hair is fixed and stabilized on hair.

A hair volume increasing agent made of a resin in liquid form for forming a hair volume increasing coating film having a large volume that has appropriate affinity and adhesiveness with a waterproof coating film is applied on the surface of the hair of the hair portion where the above described waterproof coating film is formed in such a manner that the surface of each hair is coated with a foundation agent for hair in cylindrical form so that the hair volume increasing agent is applied to the hair, and the hair is rubbed together or loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a foundation agent and a hair volume increasing agent in cylindrical form, and thus, the hair volume increasing agent is fixed and stabilized on the hair and/or the foundation agent, the above described hair volume increasing agent made of a resin in liquid form is applied to the hair on the hair portion where the above described hair volume increasing coating film is formed in order to further enhance the effects of increasing hair volume, if necessary, the hair is rubbed together with hands or the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a hair volume increasing agent in cylindrical or sheath form, and thus, the above described hair volume increasing coating film forming operation is repeated until a desired amount of hair increase is gained so that a desired amount of hair increase is gained.

A film coating agent for forming a waterproof coating film having appropriate affinity and adhesiveness with the above described hair volume increasing coating film is applied to the hair on the head portion in such a manner that the surface of each hair is coated with a foundation agent for hair and a hair volume increasing agent in cylindrical or sheath forming and the hair is rubbed together with hands or loosened with a comb or a brush until the strands of hair to which the film coating agent has been applied are separated from each other and the film coating agent solidifies, making combing smooth, and thereby, a film coating is formed on the surface of each hair which is coated with a film coating agent in cylindrical form so that a film coating agent is fixed and stabilized on hair, and thereby, the effects of increasing hair volume through strengthening and firming are provided to hair.

An additional important point of the present invention is described in the following. The surface of each hair is coated with a foundation agent in cylindrical or sheath form through treatment that is carried out in accordance with the above described method, and the foundation agent forms a coating film which is compatible with hair, has flexibility and is highly waterproof, and therefore, a firm foundation agent which does not come off when washing hair can be gained. As previously mentioned, the foundation agent is a film coating agent for forming a coating film which is compatible with hair, has flexibility and is highly waterproof, and therefore, at the point in time when the surface of hair is coated with the foundation agent in cylindrical form, the entirety (sheath form) or a portion (cylindrical form) of hair is coated with a coating film in cylindrical form which is highly waterproof so as not to come off when washing hair from the vicinity of the hair root to the tip of hair.

In Figs. 5-1 to 6-2, examples are illustrated where a hair volume increasing agent is coated on a foundation agent in cylindrical form, which is almost uniform, and in normal form, and a case is described in reference to Figs. 8-1 and 8-2 where a hair volume increasing agent is unevenly applied to hair in such a manner that only the hair volume increasing agent does not form a complete cylindrical form.

Figs. 8-1 and 8-2 are conceptual diagrams showing a hair portion in the case where a hair volume increasing agent 3 is applied unevenly so that a large amount gathers on one side of the surface of a foundation agent 2 of hair that has been treated with the foundation agent 2, as shown in Figs. 4-1 and 4-2, where Fig. 8-1 is a longitudinal cross sectional diagram showing a hair portion on the head, and Fig. 8-2 shows a cross section of the hair cut at point A-B of Fig. 8-2 as well as the appearance of hair above A-B.

As described above, treatment is carried out using a hair volume increasing agent following a foundation agent where the hair volume increasing agent is made of a resin in liquid form having appropriate affinity and adhesiveness with the waterproof coating film, which is made of the above described foundation agent, and therefore, even in the case where the hair volume increasing agent is unevenly applied to hair in such a manner that a complete cylindrical form is not formed, the hair volume increasing agent is integrated with the foundation agent, as shown in Figs. 8-1 and 8-2 (the integrated portion is the portion X shown as cross hatching in the figures: X indicates a region where agents bond), as long as the hair volume increasing agent is applied and firmly adhered to the foundation agent which forms a normal coating film in cylindrical form. That is to say, as shown in Figs. 8-1 and 8-2, the foundation agent 2 and the hair volume increasing agent 3 work as almost one resin in the region where agents bond X. In this manner, the same results are gained as in the case where hair is coated with the hair volume increasing agent in cylindrical form so that the effect is gained where the coating film which is highly waterproof does not come off when washing hair.

The same can be said in the case of a film coating agent. Figs. 6-1 and 6-2 show the state of hair 1 where hair treated with the hair volume increasing agent 3, after Figs. 5-1 and 5-2, is further treated with a film coating agent 4 by implementing the present invention. The film coating agent 4 also forms a waterproof coating film which has excellent affinity and adhesiveness with the above described hair volume increasing film, and therefore, even in the case where the film coating agent 4 is unevenly applied to hair 1 and does not form a complete cylindrical form, the same effects can be gained as those of a coating film with which hair is coated in cylindrical form and is highly waterproof so as not to come off when washing hair as long as the foundation agent and/or the hair volume increasing agent form a normal coating film in cylindrical form (though the portion corresponding to the region where agents bond is not particularly shown as cross hatching in order to prevent the figures from becoming complicated, the portion where the respective agents make contact and are connected with each other becomes the region where agents bond X, and therefore, the whereabouts of this are shown as X in Figs. 5-1 to 6-2). Here, in the case where the hair volume increasing agent 3 is not completely coated with the film coating agent 4, the effects of the film coating agent 4 of protecting the hair volume increasing agent 3 are naturally lacking in the portion where the hair volume increasing agent 3 is not completely coated. However, the hair volume increasing agent 3 of the present invention is waterproof, and therefore, the effect of increasing hair volume is not inferior even in the case where there is a portion where the film coating agent 4 is slightly lacking as described above.

That is to say, the foundation agent, the hair volume increasing agent and the film coating agent have appropriate affinity and adhesiveness with each other and are waterproof coating films, as in the portion X shown in Figs. 5-1 to 6-2, and therefore, are integrated in the portion of the region where agents bond X, forming a lump of a coating film made of almost one resin, and as a result, the coating films in cylindrical form become as if they are formed of one resin. As previously mentioned, even in the case where the hair volume increasing agent 3 and the film coating agent 4 do not form a complete cylinder on hair 1, the hair volume increasing agent 3 is integrated with the foundation agent 2, and the film coating agent 4 is integrated with the hair volume increasing agent 3 and/or the foundation agent 2 in such a manner that the same effects can be gained as in the case where the hair volume increasing agent and the film coating agent respectively form a coating film in cylindrical form as long as the hair 1 is coated with the foundation agent 2 in cylindrical form having an appropriate length, and the hair volume increasing agent 3 and the film coating agent 4 are applied and firmly adhered to the foundation agent which forms a coating film in cylindrical form. In addition to the above, even in the case where the foundation agent 2 is finished in incomplete cylindrical form, a hair volume increasing agent which provides semi-permanent effects of increasing hair volume can be gained as long as the hair volume increasing agent 3 and the film coating agent 4 are added in such a manner as to be firmly adhered to the respective agent, and thereby, the hair 1 can be totally coated with a cylinder having an appropriate length.

Next, "how each of a large number of hairs can be coated with a foundation agent for hair in cylindrical form in a short period of time when the present invention is implemented" is described using an example where hair is treated with a foundation agent in the following. "A sufficient amount of a foundation agent for soaking hair is applied to all of the hair on a head portion where thin hair grows, and it is desired that hair is increased in thickness, hair is rubbed together and/or hair is loosened using a comb or a brush so that the foundation agent for hair is applied to all of the hair from the portion of hair on the scalp to the tip of hair, and hair is loosened with a comb or a brush or is loosened with hands until the strands of hair, to which the foundation agent for hair has been applied, are separated from each other, and the foundation agent solidifies, making combing smooth, so that the foundation agent for hair forms a cylinder on the surface of each hair from the vicinity of the hair root to the tip of hair", and what is important here is the properties of the agents used in the present invention for providing a coating film which (1) is compatible with hair, (2) has flexibility, (3) has small malleability and (4) is highly waterproof. First, the foundation agent that is applied to hair (1) easily forms a coating film in cylindrical form on the surface of hair due to its compatibility with hair. Next, (2) a flexible coating film in cylindrical form can be formed without causing cracking in the resin when hair is bent due to its flexibility. What is furthermore important is that (3) the resin in the portions where the resin is finished to have a certain degree of thickness, that is to say, in the portions where the resin stiffens between strands of hair which cross and are in a gap formed between overlapping strands of hair is easily broken or cracked so that the strands of hair are separated from each other when the bundle of hair is loosened by being rubbed together or impact is provided to the hair through patting, combing or brushing.

The reason why the strands of hair can be separated from each other is described in the above, and furthermore, the relationship between the scalp, hair and the treatment agent when hair is treated with a foundation agent or the like is described in the following in reference to the drawings. Figs. 7-1 and 7-2 are conceptual diagrams for illustrating the role of the foundation agent and the theory of treatment, and Figs. 7-1 and 7-2 are longitudinal cross sectional diagrams showing hair and the scalp in a state where a foundation agent 2 of the present invention is applied to hair 1 in cylindrical form. That is to say, a sufficient amount of a foundation agent for hair, which is made of a resin in liquid form for forming a coating film that is compatible with hair, has flexibility, has small malleability and is highly waterproof, for soaking hair is applied to all of the hair on a head portion where hair grows, and it is desired that hair is increased in thickness, hair is rubbed together and/or hair is loosened using a comb or a brush so that the foundation agent for hair is applied to all of the hair from the portion of hair on the scalp to the tip of hair, and hair is loosened with a comb or a brush or hair is loosened with hands until the strands of hair are separated from each other, and the foundation agent solidifies, making combing smooth, so that the foundation agent for hair is fixed on the surface of each hair from the vicinity of the hair root to the tip of hair as a coating film in cylindrical form which is compatible with hair, has flexibility and is highly waterproof. As for the relationship between the scalp, hair and the treatment agent, as shown in Figs. 7-1 and 7-2, a sufficient amount of foundation agent 2 for soaking hair is applied onto the scalp H, and therefore, the foundation agent 2 forms a coating film in cylindrical form starting from the vicinity of the hair root M (the coating film portion in cylindrical form is indicated as T). Since hair is soaked with an amount of the foundation agent 2 which is sufficient enough for the foundation agent to enter into the hair root portion F from the vicinity of the hair root M, a coating film of the foundation agent 2 naturally attaches to the surface of the scalp H, as shown in Fig. 7-2, immediately after the operation, and as for the relationship between the foundation agent 2 and the surface of the scalp H, the foundation agent 2 does not form a cylindrical form or a sheath form when being applied to the surface of the scalp H, and therefore, most of the foundation agent 2 in this portion (portion that is attached to the scalp H) is peeled off and removed when hair is brushed with a comb or a brush when the foundation agent is dried and fixed, or even if it is not peeled off at this time, the foundation agent can be easily removed and washed off when washing hair. Therefore, finally, hair where the foundation agent 2 is fixed and stabilized only on hair 1 is done, as schematically shown in Fig. 7-1. In the above described manner, when the hair volume increasing agent 3 and the film coating agent 4 are attached to and made to overlap with the foundation agent 2 or when the hair volume increasing agent and the film coating agent each attach to the surface of the scalp H alone, in addition to when attaching only the foundation agent 2 on the surface of the scalp H, they are not applied to the surface of the scalp H in cylindrical form or sheath form, and therefore, easily peel off in the same manner as the above described foundation agent 2 when hair is brushed with a comb or a brush, or when washing hair. In addition, the coating films of the foundation agent, the hair volume increasing agent and the film coating agent which are finally attached to hair in cylindrical form after brushing or washing are in such a state as to clog the vicinity of the hair root M, which, at a first glance, seems to negatively affect the hair root portion F. In fact, they are pulled away from the surface of the scalp when hair is loosened with a comb or a brush, creating a gap in the vicinity of the hair root M, or hair grows in such a manner that the coating film in cylindrical form that is made on the hair is pulled in the direction in which the hair grows, and as a result, a complete gap is created in the vicinity of the hair root M, aerating the hair portion F. That is to say, the coating film in cylindrical form in the vicinity of the hair root M and the coating film of resin which stays in the vicinity of the hair root M do not cause any problems. In the above described manner, a great number of hairs can be treated in a short period of time, so that the strands of hair are separated from each other.

Here, the principle behind the foundation agent raising hair is described. Hair in the state shown in Fig. 7-1 is, at a glance, hair that is passed through a tube made of resin (coating film portion T in cylindrical form). In other words, it can be seen that hair is supported by a tube (coating film portion T in cylindrical form). In this manner, hair ends up increasing in thickness, and thin hair with little resilience on the scalp H is supported by a tube made of resin (coating film portion T in cylindrical form) of the foundation agent 2, making it difficult for it to fall over. That is to say, hair is raised.

As for the foundation agent for forming a coating film of the present invention which is compatible with hair, has flexibility, has little malleability and is highly waterproof, it is at the least necessary for the coating film to be in cylindrical form and work to support hair, and in order to achieve this, the solution having the foundation agent components (excluding the spraying agent) may contain a solid resin in a range from 0.5 wt % to 45 wt %, preferably 3 wt % to 35 wt % (the rest is a diluting solvent and other components). In addition, the same resin as the resin for the waterproof film coating agent which forms a coating film used in the hair volume increasing agent or film coating agent of the present invention can be used as the resin in the foundation agent.

As for the resin for a waterproof film coating agent used in the foundation agent, the hair volume increasing agent and the film coating agent of the present invention, agents which work to form a coating and applied film in cylindrical form on the surface of hair, raise hair, make the hair volume increasing component adhere and protect or hold the hair volume increasing component and the effects of increasing hair volume are used in order to protect hair by forming a waterproof coating film on hair and prevent the respective agents, for example the hair volume increasing component, from coming off from the hair under rain or a shower, or when washing hair. In particular, it is desirable for the resin for the waterproof film coating agent to prevent water from entering from the outside, prevent coming off or swelling of the hair volume increasing component, and have excellent appearance in terms of smoothness of combing at the time of setting and gloss. Taking this into consideration, a resin that is highly waterproof may be selected from among resins used as conventional setting agents for hair as the resin for the waterproof film coating agent. Unfortunately, however, the components which are used as conventional setting agents for hair originally satisfy the conditions of having excellent properties in terms of being removable through washing, in addition to having excellent setting properties and causing no loss of shape (having excellent resistance to moisture), and therefore, most resins for conventional setting agents for hair cannot be used, and only some resins for conventional cosmetics can be used as they are or modified.

Waterproof resins which can be used for the foundation agent, the hair volume increasing agent and the film coating agent of the present invention are resins which provide waterproof properties after being dried, can be used for the human body and is applicable to hair, and therefore, it is appropriate to use a resin for forming a coating film which is compatible with hair and has flexibility and little malleability, and thus, as typical waterproof resins that can be used for the foundation agent, the hair volume increasing agent and the film coating agent of the present invention, non-neutralizing copolymers of octyl amide acrylate and ester acrylate, non-neutralizing copolymer liquids of alkyl ester acrylate and diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl acrylamide acrylate, hydroxypropyl acrylate and butyl amino ethyl methacrylate, and non-neutralizing polymers of carboxyl modified vinyl acetate, in addition to urethane resins, silicone resins, urethane modified resins and silicone modified resins, can be cited. It is possible to use these resins alone or to combine two or more, and in addition, it is possible to use a copolymer of these resins with another substance (non-neutralizing in the above description means resins which are not yet neutralized). However, any resin other than those illustrated in the above can be used, as long as it is a resin which becomes waterproof after being dried, can be used for the human body, is applicable to hair and is compatible with hair, and in addition, it is possible to minutely adjust the flexibility and the malleability to within an appropriate range by adding silicone, a higher alcohol, a higher aliphatic acid, a resin powder or the like, as described below.

To repeat, it can be said that the resin components for forming a coating film which are used in the foundation agent, the hair volume increasing agent and the film coating agent of the present invention have waterproof properties in common, so that it is important for the resin components to be waterproof after being dried, and it is absolutely necessary for the film coating agent to have such properties. In addition, the foundation agent, the hair volume increasing agent and the film coating agent must have excellent adhesiveness and stickiness between respective resin coating films made of these. Therefore, the waterproof resins used for the foundation agent, the hair volume increasing agent and the film coating agent may use at least one resin component of the same or same type as that of another agent overlapping.

Here, flexibility and malleability, which are important factors in the above described resins, are described. Though it is best for these resins which are used to inherently have appropriate properties in terms of these factors, in reality, these agents are made by mixing and solving a variety of components, and therefore, the final product of these may have appropriate flexibility and malleability after being applied on the surface and dried, and from this point of view, the product can be prepared by adding a plastic agent, for example, when the resin does not have flexibility, or by adding a powder in solid form, for example, when the resin does not have appropriate malleability. The same can be said for the foundation agent, the hair volume increasing agent and the film coating agent, and this method can be applied for the respective agents if necessary.

In the case where the solvent of the foundation agent of the present invention has low volatility, it negatively affects usability and effects of increasing hair volume. Therefore, it is preferable for the diluted solvent used for the foundation agent to be a volatile solvent, and as the volatile solvent, a substance that can evaporate and vaporize relatively easily on hair, for example, a lower alcohol, such as ethanol or IPA, or a low molecular hydrocarbon, such as isopentane or hexane, is preferable. From among these, ethanol is most preferable, particularly taking into consideration the effects on the scalp and hair, smell, compatibility with the resin for forming a waterproof film coating agent and a coloring agent, solubility and the like.

A bulking component, a lubricant, a plasticizing agent, a surfactant, which are used in the below described hair volume increasing agent, and if necessary, an antiseptic, a sterilizer, a bacteriostat, a hair growth stimulant, a hair restorer, a dandruff preventing agent, vitamins or derivatives of vitamins, a chelating agent, a viscosity adjustor, a rust inhibitor for metal, animal or vegetable extract, an antioxidant, an anti-inflammatory agent, a blood circulation stimulant or a perfume can be mixed in with the foundation agent of the present invention, in order to increase the functionality, usability and hair raising properties at the time of treatment. Here, it is important not to decrease the original target performance for the foundation agent by adding these, and in order not to do so, it is necessary for the bulking component, which is, for example, a powder, to be no greater than 25 weight percent, for the lubricant, which is, for example, silicone, to be no greater than 15 weight percent, for the plasticizing agent to be no greater than 10 weight percent, for the surfactant and other components to be no greater than 5 weight percent, and for the total amount of solvent and the above described components other than the bulking component, for example a waterproof resin or a powder, to be 17 percent of the total amount of the foundation agent at the most. In the case where the percentage exceeds this, a foundation agent which is difficult to handle or has poor usability or poor effects as a foundation is generally gained.

It is most preferable to finish a hair volume increasing agent using only a resin having bulking properties as the hair volume increasing component in the present invention. The reason for this is that such a resin easily provides a natural feel on the surface of the hair after treatment, which is not different from natural hair, when the present invention is implemented. From this point of view, it is also possible to use a small amount of resin having high effects on bulking properties, and to treat hair many times with the hair volume increasing agent.

However, it is, of course, better for the hair volume increasing agent to have great effects of increasing hair volume when the present invention is implemented, and thus, the following hair volume increasing agent is provided, which makes the effects of increasing hair efficient when used in accordance with the present invention.

The hair volume increasing agent may be of a one-liquid type where the hair volume increasing agent contains at least both the hair volume increasing component and the resin for the waterproof film coating agent, or may be formed of, for example, two liquids used in two processes where a hair volume increasing component, which is a short fiber substance or a powder, is electrostatically applied to hair, and after that, a resin liquid having a fixing component is applied while paying attention so that the hair volume increasing component does not come off, and thus, the hair volume increasing component is fixed on the hair in the case where it is possible to comb the hair after the hair volume increasing component is applied, and then, the resin for the waterproof film coating agent is applied and fixed. As the hair volume increasing component, any of a variety of types, for example a hair volume increasing component having such a size that it can adhere to hair, for example a hair volume increasing component having pseudo-effects of increasing hair volume, such as a pigment and/or a pigment bulking agent used in conventional hair colors, a powder in solid form or other substances in powder form, such as a short fiber substance, can be used in the present application. Any one type from among the following, or a mixture of any of the following can be used as the above described hair volume increasing component: pigments such as yellow titanium oxide, red iron oxide, black iron oxide, iron blue, chromium oxide, ultramarine blue, carbon black, umber, calamine, humic acid, and Indian ink, powder in solid form such as silicate anhydride, magnesium silicate, talc, kaolin, bentonite, serilite, titanium mica, cerium oxide, zirconium oxide, zinc oxide, titanium oxide, precipitating calcium carbonate, aluminum oxide, aluminum magnesium silicate, calcium sulfate, chalk powder, pumice powder, pearl powder, silk powder, crystal cellulose powder, whiting, wool powder, cotton fiber powder, starch, styrene foam resin powder, microscopic urea resin powder.

Furthermore, it is preferable to adopt a hair volume increasing agent as a hair volume increasing hair color by adding a coloring agent. As the coloring agent, any one type or mixture of any of the following can be adopted: dies such as tar pigment, red No. 2, brown No. 201, blue No. 203, yellow No. 402, green No. 401, purple No. 401 and black No. 401, pigments such as yellow titanium oxide, red iron oxide, black iron oxide, coloring iron oxide (color iron oxide), ultramarine blue, iron blue, chromium oxide, carbon black, umber, calamine, humic acid and Indian ink, and natural pigments, such as carotene, quercetin, laccaic acid, chlorophyllin, betanin, cocoa pigment, curcumin, carminic acid, globulin, squid ink, berberine chloride, riboflavin, copper chlorophyllin Na, hematine, hematin, azulene.

As described above, it is most preferable to use a resin having excellent bulking properties by itself to finish the hair volume increasing agent which is used in the present invention. However, it is, of course, most effective to use other hair volume increasing components together in order to gain the effects of increasing hair volume. Thus, in terms of the components of the hair volume increasing agent, it is preferable for the resin for a waterproof film coating agent to be 99.9 weight parts to 40 weight parts relative to 0.1 weight parts to 60 weight parts of the solid component (including the case where a coloring agent is contained), which is the above described hair volume increasing component, and it is more preferable for 0.5 weight parts to 35 weight parts of a solid and 99.5 weight parts to 65 weight parts of a resin for a waterproof film coating agent to be mixed in, in order to gain a hair volume increasing agent that has excellent usability at the time of application and is easy to handle. In addition, though it depends on the resin used for the waterproof film coating agent, it is generally preferable to provide the hair volume increasing agent in the form of aerosol, in order to make drying easier, make handling easier and shorten the time for application. It is necessary to dilute the hair volume increasing agent by adding a solvent in the case where the content composite is processed for such an aerosol hair volume increasing agent. In this case, 5 weight parts to 95 weight parts of solvent may be used for 95 weight parts to 5 weight parts of the hair volume increasing agent components, as described above.

When a solvent is added to the hair volume increasing agent and the film coating agent of the present invention, the viscosity of the original liquid lowers, and thus, preferable results are gained when filling a container (polyethylene container or can for aerosol) with the original liquid, but the usability and the effects of increasing hair volume are negatively affected when the volatility of the solvent is low. Therefore, it is preferable for the solvent to be a volatile solvent, and as the volatile solvent, a substance that can evaporate and vaporize relatively easily on hair, for example, a lower alcohol, such as ethanol or IPA, or a low molecular hydrocarbon, including, such as isopentane or hexane, is preferable. From among these, ethanol is most preferable, particularly taking into consideration the effects on the scalp and hair, smell, compatibility with the resin for forming a waterproof film coating agent and a coloring agent, solubility and the like.

In order to make the usability and the effects of increasing the thickness of the film (increasing the volume) easy to gain, it is preferable for the amount for application at one time (amount of hair volume increasing agent used at one time) to be completely dried within five minutes at the slowest during treatment. This can be arbitrarily set by examining appropriate values for the mixture ratio of the component of the used hair volume increasing agent, that is to say, the solid (including the case where a coloring agent is contained) to the resin for the coating film, and the mixture ratio of the solid, the resin for the coating film and the solvent in the case where the solvent is added. It is more effective for this time for drying to be within three minutes during treatment. This is because adhesiveness on the surface is lost soon after hair is loosened with a comb, so that strands of hair do not entangle or adhere to each other, and thereby, the work of loosening with a comb or a brush becomes less, reducing the number of strokes in combing, and accordingly, the layered solid solidifies without breaking during the work of applying an appropriate amount of the above described hair volume increasing agent to hair until sufficient effects of increasing hair volume are gained and rubbing the hair together with the hands so that the hair volume increasing agent is applied to all of the hair, or lightly loosening the hair with a comb so that strands of hair do not entangle or adhere to each other when dried, and therefore, the effects of increasing hair volume are significant and the work can be carried out without the user (worker) getting tired, which is preferable.

In addition, the above described original liquid with components may contain a surfactant, a lubricant, a plasticizing agent or the like, in addition to the hair volume increasing component, the waterproof film coating agent, the coloring agent and the volatile solvent. As for the surfactant, any type of surfactant from among anionic, non-ionic, cationic and amphoteric surfactants can be adopted, as long as there are no problems in terms of compatibility with the hair volume increasing component, the adhesive resin and the coloring agent used in the above described hair volume increasing agent used as a component of the liquid with a coloring component. Here, waterproof properties tend to be poor when a large amount of surfactant is added, and therefore, it is important not to add more surfactant than necessary, and from this point of view, in the case where such a surfactant is used, it is desirable for the ratio occupied by the entirety of this surfactant to be no greater than 3 % of the total components, and it is preferable for it to be no greater than 1 %.

A lubricant is used in order to increase the spreading properties of the hair volume increasing agent after the latter is applied to hair, as well as in order to increase the smoothness of combing after being dried. As for the lubricant, any of a variety of conventional lubricating oil solutions, such as silicone with low polymerization, silicone resins and fluorine resins, can be used. It is preferable to use a hydrophobic lubricant. It is also possible to use higher alcohols, glycols and higher aliphatic acids as the plasticizing agent, or the plasticizing agent may be substituted with another surfactant, as described above, or a lubricant. Here, lubricants such as silicone with low polymerization, silicone resins and fluorine resins, as well as plasticizing agents such as higher alcohols and higher aliphatic acids, greatly affect flexibility, malleability and waterproof properties of the waterproof film coating agent, and therefore, it is necessary to pay sufficient attention to the amount used in the composition, as well as the amount of respective composites. In addition, it is preferable to use a hydrophobic plasticizing agent as the lubricant or the plasticizing agent.

Furthermore, in addition to the above, a solvent, a glossing component, an ultraviolet ray blocking agent, an antiseptic, a sterilizer, a bacteriostat, a hair growth stimulant, a hair restorer, a dandruff preventing agent, vitamins or derivatives of vitamins, a chelating agent, a viscosity adjustor, a rust inhibitor for metal, animal or vegetable extract, an antioxidant, an anti-inflammatory agent, a blood circulation stimulant or a perfume can be mixed in with the hair volume increasing agent if necessary.

As the spraying agent, liquefied gases, such as LPG, DME and fluorohydrocarbon (F134a and the like), as well as compressed gases, such as carbonic acid gas, nitrous oxide gas and nitrogen gas can be adopted. A pressure-resistant container may be filled with a spraying agent, so that the inner pressure at 35 °C comes within the range of 0.2 Mp to 0.8 Mp.

In the case where the entirety of the aerosol hair volume increasing agent is 100 wt %, it is preferable for the spraying agent described above to be less than 70 wt %, and in particular, it is preferable for the spraying agent to be 63 wt % to 68 wt %, because splashing of the hair volume increasing agent is prevented to a certain degree and drying is very easy after application.

The film coating agent of the present invention forms a coating film which is compatible with hair, has flexibility and is highly waterproof, and protects hair by forming a waterproof coating film on hair, the foundation agent and the hair volume increasing component, and in addition, works to form a coating film in cylindrical form on the surface of hair for coating and protecting hair, so that it becomes difficult for the foundation agent and the hair volume increasing components to come off from the hair due to rain or a shower, or when washing hair.

In particular, it is desirable for the film coating agent to prevent water from entering from the outside to the hair after treatment, and to prevent peeling off or swelling of the hair volume increasing component, and in addition, to provide excellent appearance in terms of smoothness of combing at the time of setting and gloss. It is important for the resin component used in the film coating agent to be waterproof after drying, and this is absolutely necessary. In addition, the resin component must have high adhesiveness and stickiness with the foundation agent and the hair volume increasing agent between the coating films of the respective resins. Therefore, at least one resin component of the waterproof resin used for the film coating agent may use the same or same type of resin component as that which overlaps with the foundation agent and the hair volume increasing agent. Here, in the same manner as the foundation agent and the hair volume increasing agent described in the above as a resin component, it is necessary for the waterproof resin to become waterproof after drying, be usable for the human body, be applicable to hair, be compatible with hair and have flexibility. From this point of view, it is preferable to use the same resin as the above described foundation agent and hair volume increasing agent. That is to say, as the waterproof resin, non-neutralizing copolymers of octyl amide acrylate and ester acrylate, non-neutralizing copolymer liquids of alkyl ester acrylate and diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl acrylamide acrylate, hydroxypropyl acrylate and butyl amino ethyl methacrylate, and non-neutralizing polymers of carboxyl modified vinyl acetate, in addition to urethane resins, silicone resins, urethane modified resins and silicone modified resins, are typically used. It is possible to use these resins alone or to combine two or more, and in addition, it is possible to use a copolymer of these resins with each other or with another substance. From this point of view, the waterproof resin may be used as a resin solid in the solution with the film coating agent component (excluding the spraying agent) in a range from 0.1 weight percent to 30 weight percent, preferably from 0. 5 weight percent to 20 weight percent.

In addition, the film coating agent in many cases works also as a setting agent, and therefore, a microscopic amount of another resin can be added in such a range that the waterproof properties are not lost, in order to enhance the setting properties. As a resin that can be used for this purpose, synthetic polymer compounds, such as polyvinyl pyrrolidone, polyvinyl alcohol, polyacrylamide, polyacrylates, acryl resin alkanolamine liquids, copolymer liquids of N-methacryloyloxyethyl N, N-dimethyl ammonium-α-N-methyl carboxybetaine and alkyl ester methacrylate, copolymers of methacryloyl ethyl dimethyl betaine, methacryloyl ethyl trimethyl ammonium chloride and 2-hydroxyl ethyl methacrylate, copolymer liquids of methacryloyl ethyl dimethyl betaine, methacryloyl ethyl trimethyl ammonium chloride and methoxy polyethylene glycol methacrylate, and copolymers of methoxyethylene and maleic anhydride, natural polymer compounds, such as carrageenan, xanthan gum, gelatin, dextrin, natural rubber, ester rubber, modified starch and milk casein, and semi-synthetic polymer compounds can be cited as examples, and the amount used is limited to such a range that the waterproof properties are not lost, and thus, is limited to no greater than 5 weight percent of the used waterproof resin, preferably no greater than 1 weight percent. Furthermore, in addition to the above, a solvent, a glossing component, an ultraviolet ray blocking agent, an antiseptic, a sterilizer, a bacteriostat, a hair growth stimulant, a hair restorer, a dandruff preventing agent, vitamins or derivatives of vitamins, a chelating agent, a viscosity adjustor, a rust inhibitor for metal, animal or vegetable extract, an antioxidant, an anti-inflammatory agent, a blood circulation stimulant or a perfume can be mixed in with the hair volume increasing agent if necessary.

In general, it is preferable to provide the film coating agent in aerosol form, in order to make drying easier, make handling easier and shorten the time for application. When providing the film coating agent in aerosol form, the usability is negatively affected if the volatility of the solvent is low. Therefore, it is preferable for the solvent of the resin for the waterproof film coating agent to be a volatile solvent, and as the volatile solvent, a substance that can evaporate and vaporize relatively easily on hair, for example, a lower alcohol, such as ethanol or IPA, or a low molecular hydrocarbon, including such as isopentane or hexane, is preferable. From among these, ethanol is most preferable, particularly taking into consideration the effects on the scalp and hair, smell, compatibility with the resin for forming a waterproof film coating agent and a coloring agent, solubility and the like.

In the case where a film coating agent in aerosol form is prepared, as the spraying agent, liquefied gases, such as DME, LPG and fluorohydrocarbon (F134a and the like), as well as compressed gases, such as carbonic acid gas, nitrous oxide gas and nitrogen gas can be adopted. A pressure-resistant container may be filled with a spraying agent, so that the inner pressure at 35 °C comes within the range of 0.2 Mp to 0.8 Mp. In the case where the entirety of the film coating agent in aerosol form is 100 wt %, 70 wt % to 10 wt % of spraying agent may be used relative to 30 wt % to 90 wt % of the film coating agent which includes the solvent.

### Examples

### Example 1

A liquid component agent having the following composition was prepared.

| foundation agent I (liquid foundation component agent) | |
|---|---|
| copolymer of vinyl acetate and crotonic acid (non-neutralized) | 10.0 wt % |
| silicone (low molecular dimethyl silicon) | 0.2 wt % |
| ethanol anhydride | 89.8 wt % |
| total | 100.0 wt % |

| hair volume increasing agent I (liquid hair volume increasing component agent) | |
|---|---|
| copolymer of vinyl acetate and crotonic acid (non-neutralized) | 18.6 wt % |
| coloring iron oxide (black) | 3.0 wt % |
| ultramicroscopic particles | |
| of silica anhydride | 0.3 wt % |
| titanium oxide | 0.1 wt % |
| ethanol anhydride | 78.0 wt % |
| total | 100.0 wt % |

| film coating agent I (liquid film coating component agent) | |
|---|---|
| copolymer of vinyl acetate and crotonic acid (non-neutralized) silicone (low molecular | 9.3 wt % |
| dimethyl silicon) | 0.2 wt % |
| ethanol anhydride | 90.5 wt % |
| total | 100.0 wt % |

The above described foundation agent was divided into plastic containers, and a sufficient amount of the foundation agent for soaking hair was taken from one of the plastic containers and applied to all of the hair on a head portion where thin hair grew, and it was desired that hair was to be increased in thickness; hair was rubbed together so that the foundation agent was applied to all of the hair; and hair was loosened with a comb or a brush or was loosened with hands by rubbing hair together until the strands of hair, to which the foundation agent had been applied, were separated from each other, and the foundation agent solidified, making combing smooth, so that the foundation agent formed a coating film in cylindrical form on the surface of each hair from the vicinity of the hair root to the tip of hair, and thus, the foundation agent for hair was fixed to and stabilized on hair before being increased in thickness.

The above described hair volume increasing agent was divided into polyethylene containers with a spray pump, and the hair volume increasing agent was sprayed little by little onto hair so as to be applied to hair on a head portion where a waterproof coating film was formed in such a manner that the surface of each hair was coated with a foundation agent for hair in cylindrical form in the above described process for fixing and stabilizing a foundation agent for hair onto hair while the hair was being rubbed together so that the hair volume increasing agent was applied to hair, and hair was loosened with a comb or a brush until the strands of hair, to which the hair volume increasing agent had been applied, were separated from each other, and the hair volume increasing agent solidified, making combing smooth, and thus, a hair volume increasing coating film was formed on the surface of each hair in such a manner that hair was coated with the foundation agent and the hair volume increasing agent in cylindrical form, and the hair volume increasing agent was fixed to and stabilized on the hair together with the foundation agent with this treatment being expanded throughout the entirety of the hair on the head portion where thin hair grew, and it was desired that hair was to be increased in thickness.

In order to further increase the effects of increasing hair volume, the hair volume increasing agent was subsequently sprayed little by little from one of the above described polyethylene containers with a spray pump, into which the hair volume increasing agent had been divided, onto hair so that the hair volume increasing agent was applied to hair on the head portion where the above described hair volume increasing coating film had been formed, and then, hair was loosened with a comb or a brush or hair was rubbed together with hands until the strands of hair, to which the hair volume increasing agent had been applied, were separated from each other, and the hair volume increasing agent solidified, making combing smooth, so that a hair volume increasing coating film was formed on the surface of each hair so that each hair was coated with the hair volume increasing agent in cylindrical form, and thus, the above described hair volume increasing coating film forming operation was repeated three times until a desired amount of hair volume increase was achieved.

In the process of repeating the above described hair volume increasing coating film forming operation, a film coating agent was sprayed little by little from one of the polyethylene containers with a spray pump, into which the film coating agent had been divided, onto all of the hair where the surface of each hair was coated with the hair volume increasing agent together with the foundation agent in cylindrical form so that a desired amount of hair increase was achieved, and applied to the hair after the hair volume increasing coating film had been formed, and hair was loosened with a comb or a brush, or hair was rubbed together with hands until the strands of hair, to which the film coating agent had been applied, were separated from each other, and the film coating agent solidified, making combing smooth, and thereby, a film coating was formed on the surface of each hair so that each hair was finally coated with the film coating agent in cylindrical form, and thus, the film coating agent was fixed to and stabilized on hair.

After that, hair was sufficiently dried using a dryer or the like, and then hair was washed with warm water and the shampoo of the below described Table 1 followed by setting to a desired hairstyle.

### Example 2

A liquid component agent having the following composition was prepared.

| foundation agent II (liquid foundation component agent) | |
|---|---|
| polymer of carboxyl modified vinyl acetate (non-neutralized) | 7.0 wt % |
| copolymer of vinyl acetate and crotonic acid (non-neutralized) | 8.0 wt % |
| silicone (low molecular dimethyl silicon) | 0.2 wt % |
| higher aliphatic acid | 0.3 wt % |
| ethanol anhydride | 84.5 wt % |
| total | 100.0 wt % |

The above described liquid foundation component agent was divided into polyethylene containers, and each division was used as a foundation agent.

| hair volume increasing agent II (liquid hair volume increasing component agent) | |
|---|---|
| polymer of carboxyl modified vinyl acetate (non-neutralized) | 23.6 wt % |
| coloring iron oxide (black) | 5.0 wt % |
| ultramicroscopic particles of silica anhydride | 0.5 wt % |
| titanium oxide | 0.1 wt % |
| ethanol anhydride | 70.8 wt % |
| total | 100.0 wt % |

The above described liquid hair volume increasing component agent was put into a pressure-resistant container, and after that, a valve was installed and engaged, and then, dimethyl ether was put through the valve under pressure so that the container was filled in with dimethyl ether in a ratio of 33 wt % of the liquid hair volume increasing component agent to 67 wt % of dimethyl ether, and thus, an aerosol product was prepared. This was used as a hair volume increasing agent aerosol.

| film coating agent II (liquid film coating component agent) | |
|---|---|
| polymer of carboxyl modified vinyl acetate (non-neutralized) | 11.0 wt % |
| liquid copolymer of alkyl ester acrylate, diacetone acrylamide and methacrylic acid (non-neutralized) | 1.5 wt % |
| silicone (low molecular dimethyl silicon) | 0.5 wt % |
| ethanol anhydride | 87.0 wt % |
| total | 100.0 wt % |

The above described liquid film coating component agent was put into a pressure-resistant container, and after that, a valve was installed and engaged, and then, a mixed spraying agent where dimethyl ether and a liquefied petroleum gas were mixed in a ratio of 90 wt % to 10 wt % was put through the valve under pressure so that the container was filled in with the spraying agent in a ratio of 30 wt % of the liquid film coating component agent to 70 wt % of the spraying agent, and thus, an aerosol product was prepared. This was used as a film coating agent aerosol.

The foundation agent of Example 2 was used in the same manner as in Example 1, and the above described foundation agent was divided into polyethylene containers, and a sufficient amount of the foundation agent for soaking hair was taken from one of the polyethylene containers and applied to all of the hair on a head portion where thin hair grew, and it was desired that hair was to be increased in thickness; hair was rubbed together so that the foundation agent was applied to all of the hair; and hair was loosened with a comb or a brush or was loosened with hands by rubbing hair together until the strands of hair, to which the foundation agent had been applied, were separated from each other, and the foundation agent solidified, making combing smooth, so that the foundation agent formed a coating film in cylindrical form on the surface of each hair from the vicinity of the hair root to the tip of hair, and thus, the foundation agent for hair was fixed to and stabilized on hair before being increased in thickness.

The above described hair volume increasing agent aerosol was sprayed onto hair so as to be applied to hair on a head portion where a waterproof coating film was formed in such a manner that the surface of each hair was coated with a foundation agent for hair in cylindrical form in the above described process for fixing and stabilizing a foundation agent for hair onto hair while the hair was being rubbed together in the left, right, front and rear directions with the hand which did not hold the hair volume increasing agent aerosol, paying attention so that the spray of the hair volume increasing agent aerosol was not applied to the hand, and thus, the hair volume increasing agent was applied to all of the hair where it was desired to increase hair in thickness, and after that, hair was loosened with a comb or a brush, or hair was rubbed together with one or both hands until the strands of hair, to which the hair volume increasing agent had been applied, were separated from each other, and the hair volume increasing agent solidified, making combing smooth, and thus, a hair volume increasing coating film was formed on the surface of each hair in such a manner that hair was coated with the foundation agent and the hair volume increasing agent in cylindrical form.

In order to further increase the effects of increasing hair volume, the above described hair volume increasing agent aerosol was subsequently sprayed onto hair so that the hair volume increasing agent was applied to hair on the head portion where the above described hair volume increasing coating film had been formed, and then, hair was loosened with a comb or a brush or hair was rubbed together with hands until the strands of hair, to which the hair volume increasing agent had been applied, were separated from each other, and the hair volume increasing agent solidified, making combing smooth, so that a hair volume increasing coating film was formed on the surface of each hair so that each hair was coated with the hair volume increasing agent in cylindrical form, and thus, the above described hair volume increasing coating film forming operation was repeated three times until a desired amount of hair volume increase was achieved.

In the process of repeating the above described hair volume increasing coating film forming operation, a film coating agent aerosol was sprayed onto all of the hair where the surface of each hair was coated with the hair volume increasing agent together with the foundation agent in cylindrical form so that a desired amount of hair increase was achieved, and applied to the hair after the hair volume increasing coating film had been formed, and hair was rubbed together in the left, right, front and rear directions with the hand which did not hold the film coating agent aerosol, paying attention so that the spray of the film coating agent aerosol was not applied to the hand, so that the film coating agent was applied to all of the hair where it was desired that hair was to be increased in thickness, and after that, hair was loosened with a comb or a brush, or hair was rubbed together with one or both hands until the strands of hair, to which the film coating agent had been applied, were separated from each other, and the film coating agent solidified, making combing smooth, and thereby, a film coating was formed on the surface of each hair so that each hair was finally coated with the film coating agent in cylindrical form, and thus, the film coating agent was fixed to and stabilized on hair.

After that, hair was sufficiently dried using a dryer or the like, and then hair was washed with warm water and the shampoo of Table 1 followed by setting to a desired hairstyle.

Figs. 9 to 16 are recording photographs instead of diagrams, showing the examples of the present invention, and Photographs 1 to 8 exhibited in Figs. 9 to 16, respectively, record appearances where Example 2 was used. Photograph 1 shows a state of hair before implementation and after hair was washed, dried and set as usual by the user; Photograph 2 shows a state of hair in which a sufficient amount of the foundation agent for soaking hair was applied from one of the polyethylene containers to which the foundation agent had been divided; Photograph 3 shows a state where hair was being rubbed together with hands and loosened so that the foundation agent was applied to all of the hair; Photograph 4 shows a state where the volume of hair was being increased by using the hair volume increasing agent aerosol on the surface of hair that had been coated with the foundation agent in cylindrical form; Photograph 5 shows a state where hair was being loosened with a comb until the strands of hair is separated from each other, the film coating agent solidifies, making combing smooth, after the film coating agent had been applied after the treatment with the foundation agent and the hair volume increasing agent; Photograph 6 shows a state where hair was being washed with a shampoo after treatment with the foundation agent, the hair volume increasing agent and the film coating agent; Photograph 7 shows a state where hair was being set to the hairstyle desired by the user after hair was washed and dried; and Photograph 8 shows a state of hair of the user after the hair volume was increased and setting was completed. It can be clearly seen from Photographs 7 and 8 that the hair volume of the user was increased. The recording photographs of Photographs 1 to 8 are pictures taken from the recorded video, and therefore, there are defects in that the image quality is rough and portions to be compared do not completely coincide, but it can be clearly seen that effective hair volume increasing treatment was carried out simply and easily with a hair volume increasing agent which is waterproof and resistant when being washed.

Next, liquid component agents having the compositions in the below described Table 1 were prepared for Examples 3 to 5, and liquid component agents having the compositions in the below described Table 2 were prepared for Comparative Examples 1 to 5, and each of these was used as a foundation agent, a hair volume increasing agent or a film coating agent when each example was implemented. Here, the methods for carrying out treatment on hair in Examples 3 to 5 and Comparative Examples 1 to 5 all followed the method in Example 2.

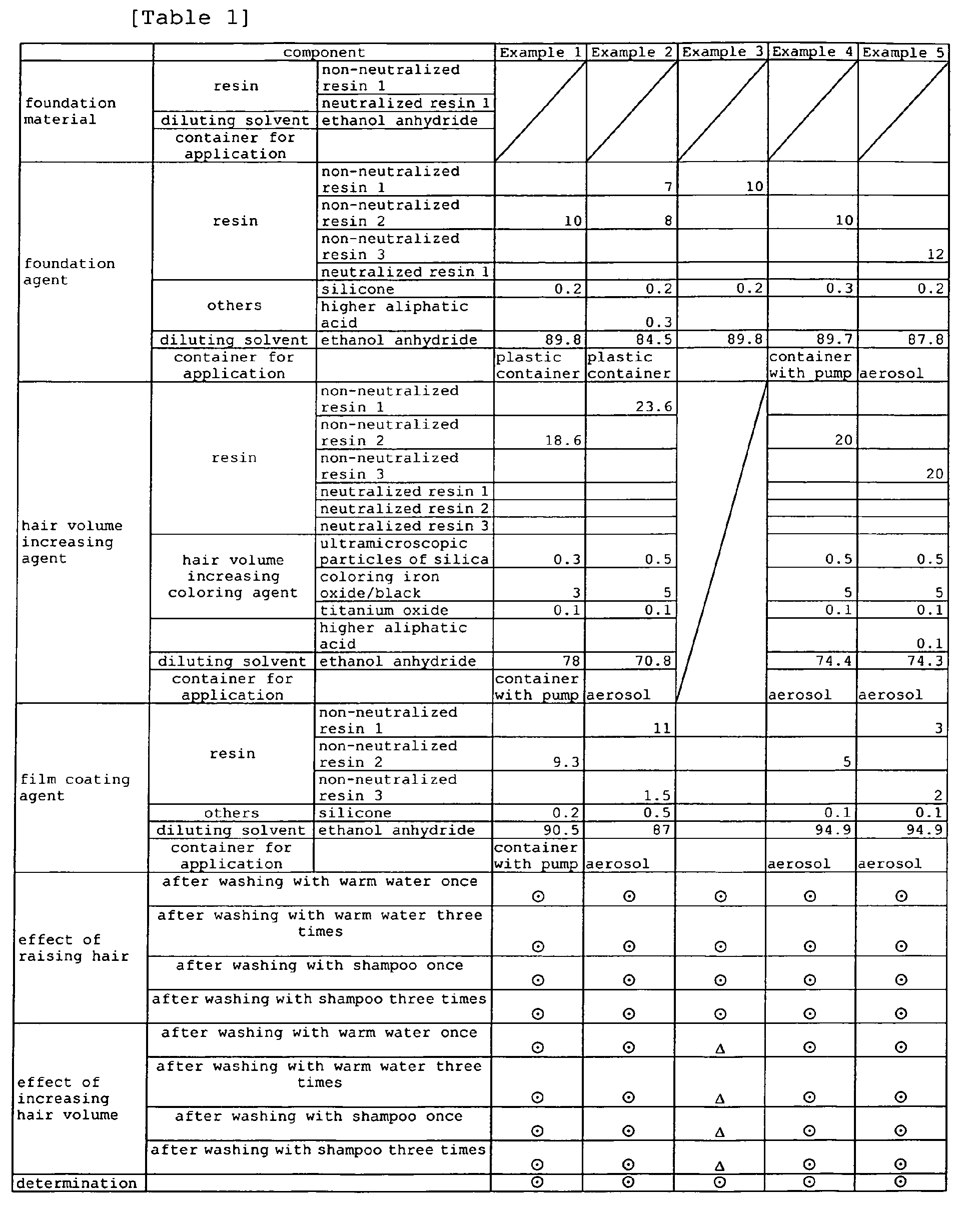

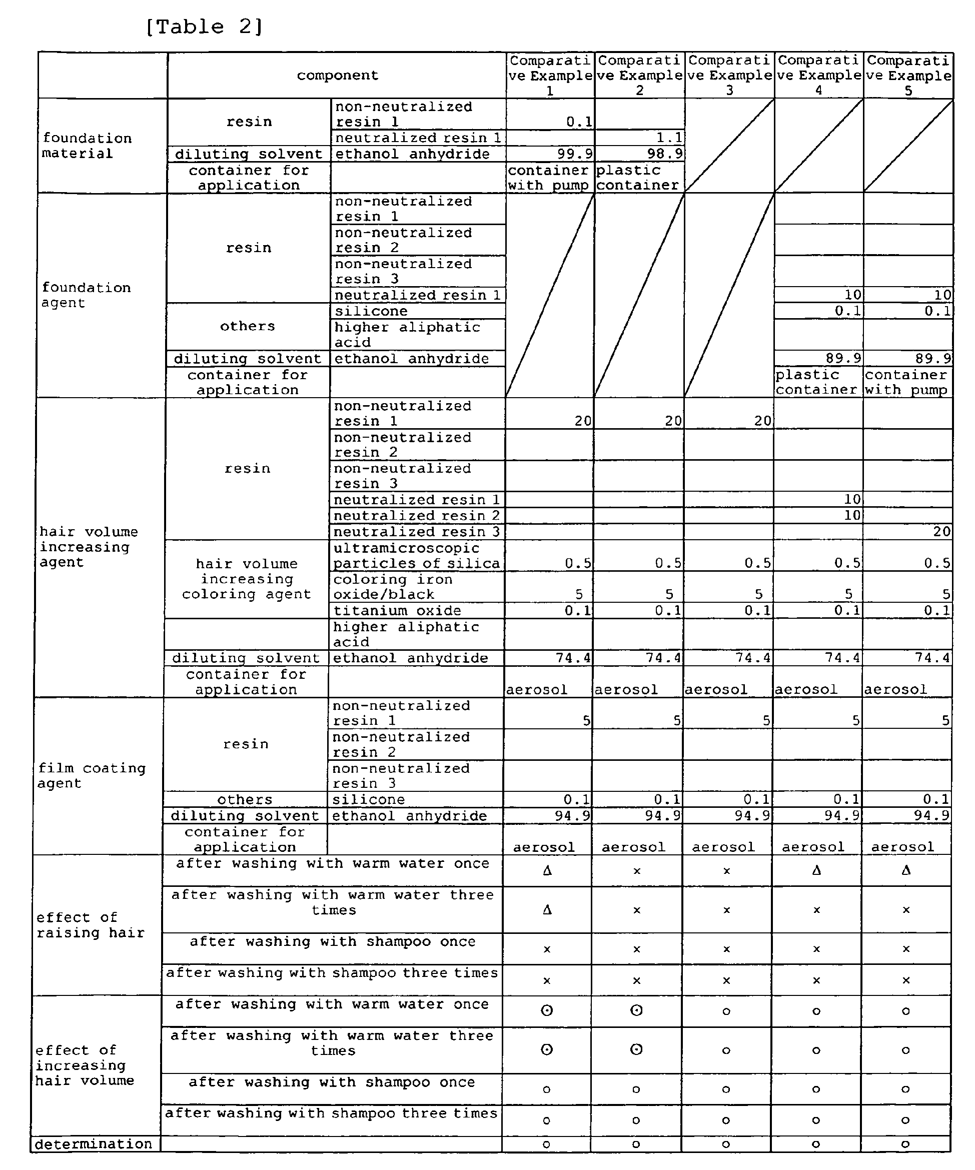

In addition, the components of Examples 1 and 2 as well as the determination results are described in Table 1.

The effects of raising hair and the effects of increasing hair volume were confirmed as shown in Tables 1 and 2 after three hours had passed after hair was treated with Examples 1 to 5 and Comparative Examples 1 to 5, respectively, after washing hair with warm water once, after washing hair with warm water three times, after washing hair with a shampoo once and after washing hair with a shampoo three times, respectively. Here, the following conditions were set and implemented when the descriptions and the effects in Tables 1 and 2 were confirmed. [Concerning names of resins in Tables 1 and 2]

The resins from among the components in the treatments in Tables 1 and 2 have long names with many letters, of which the description needs a large table, and therefore, the resins are represented with the following provisional names for the purpose of making these easy to read.
(1) Non-neutralized resin 1: non-neutralized polymer of carboxyl modified vinyl acetate (unneutralized)
(2) Non-neutralized resin 2: non-neutralized copolymer of vinyl acetate and crotonic acid (unneutralized)
(3) Non-neutralized resin 3: non-neutralized copolymer liquid of alkyl ester acrylate, diacetone acrylamide and methacrylic acid (unneutralized)
(4) Neutralized resin 4: neutralized polymer of carboxyl modified vinyl acetate
(5) Neutralized resin 5: neutralized copolymer of vinyl acetate and crotonic acid
(6) Neutralized resin 6: neutralized copolymer liquid of alkyl ester acrylate, diacetone acrylamide and methacrylic acid

Here, in the above description "non-neutralized resin" means being "non-neutralized in a state of resin or even only resin solution" whether the resin is of a type where it is general to use after neutralization or instead the resin is used without being neutralized and also means that finally "the resin is used in the component liquid in a state of not being neutralized."

In addition, "neutralized resin" means not only the "neutralized resin" where the resin is neutralized using a neutralizer at a stage where the resin solely exists, but also the "resin that is used in a state of being finally neutralized in the component liquid" including a case where the resin is finally neutralized by adding a necessary amount of neutralizer to this resin during the process for finishing the component liquid that includes the resin. Therefore, the amount of the resin of the "neutralized resin" shown in the table is the total amount of the resin and the neutralizer.

The plastic container shown in the column of container for application in Tables 1 and 2 is one of the bottles made of plastic into which the liquid component agent is divided as is, the container with a pump shown in the column is one of the bottles with a spray pump made of plastic into which the liquid component agent is divided as is, and the aerosol shown in the column is each liquid component agent which has been converted to aerosol as follows. As for the foundation agent, an appropriate amount of that liquid component agent for the foundation agent was put into a pressure-resistant container, and then, a valve was installed and engaged, and after that, a nitrogen gas was put through the valve under pressure so that the container was filled in under 0.7 mega pascal, and thus, an aerosol product was gained and used as a foundation agent aerosol. As for the liquid hair volume increasing component agent, the liquid component agent for the hair volume increasing agent and dimethyl ether were put into a pressure-resistant container so that the ratio of the liquid component agent for the hair volume increasing agent to dimethyl ether became 33 wt % to 67 wt %, and then, a valve was installed and engaged, and after that dimethyl ether was put through the valve under pressure so that the container was filled in and an aerosol product was gained and used as a hair volume increasing aerosol. As for the film coating agent, a liquid film coating component agent was put into a pressure-resistant container so that the ratio of the liquid component agent of the film coating agent to the spraying agent became 30 wt % to 70 wt %, and then, a valve was installed and engaged, and after that a mixed spraying agent of dimethyl ether and a liquefied petroleum gas in the ratio of 90 wt % to 10 wt % was put through the valve under pressure so that the container was filled in, and thus, a aerosol product was gained. This is used as a film coating agent aerosol.

### [Concerning Terms in Table 1 et cetera]

(1) warm water: to wash hair using warm water at a temperature of 35 degrees to 45 degrees
(2) shampoo: to shampoo hair using an item that is prepared in accordance with the description in the following
(3) washing hair with warm water and drying: hair is washed with warm water while lightly loosening hair with fingers for approximately three minutes, and after that, hair is dried gently with a towel and then hair is naturally dried and finished
(4) washing hair with shampoo and drying: hair is washed with warm water as described above while lightly loosening hair with fingers for approximately two minutes, and next hair is washed with a shampoo while loosening hair with a brush for washing hair having coarse bristles for approximately three minutes, and after that, lightly washed with warm water and again hair is washed with a shampoo while lightly loosening hair with a brush for washing hair having coarse bristles for approximately two minutes. After that, hair is washed with warm water for approximately three minutes, and hair is dried gently with a towel and is naturally dried so as to be finished.

The ingredients of the shampoo used for the test were as follows.

| | |
|---|---|
| (1) amido propyl betaine laurate | 30.00 wt % |
| (2) POE (2) sodium lauryl ether sulfate | 2.00 wt % |
| (3) N-lauroyl-N-methyl-β-sodium alanine | 6.00 wt % |
| (4) palm oil aliphatic diethanolamide | 2.50 wt % |
| (5) PCA-sodium | 2.00 wt % |
| (6) disodium edetate | 0.05 wt % |
| (7) purified water | 33.05 wt % |
| (8) lactic acid (solution of 17.7 %) | microscopic amount |
| (9) chitosan pyrrolidone carboxylate (solution of 1 %) | 20.20 wt % |
| (10) 1, 3 butylene glycol | 4.00 wt % |
| (11) methyl parahydroxybenzoate | 0.20 wt % |
| total | 100.00 wt % |

(11) was heated and dissolved in (10), and after that, (1) to (7) were added, and the mixture was stirred and cooled to 40 degrees and was adjusted to PH 5.8 with (8) and then (9) was added so as to gain a shampoo.

### [Effects and Determination]

(1) Effects of raising hair
   ⊙: it is clearly seen that hair is raised
   o: it can be seen that some hair is raised
   Δ: it is felt that hair is slightly raised
   ×: it is not felt at all that hair is raised
(2) Effects of increasing hair
   ⊙ : it is clearly seen that hair volume is increased
   o: it can be seen that hair volume is moderately increased
   Δ : it is felt that hair volume is slightly increased
   ×: it is not felt at all that hair volume is increased
Results: Example 3 is an example where the effects of raising hair and the effects of increasing hair volume of the present invention are shown in the case where only the foundation agent was used and Examples 4 and 5 are examples in the case where a resin which is different from those of Examples 1 and 2 was used. In all of Examples 1, 2, 4 and 5, though partially colored pieces were seen floating in warm water, the hair volume increasing agent was not eluted into warm water. In addition, the effects of raising hair and/or the effects increasing hair volume were also maintained. In addition, in Example 3, significant effects of raising hair were seen and the effects of increasing hair volume were also slightly exhibited. Comparative Example 1 from among Comparative Examples 1, 2, 3, 4 and 5 is a comparative example in the case where a polymer of carboxyl modified vinyl acetate (non-neutralized) which is waterproof was used, a foundation material made of a resin having an appropriate concentration to be used for a foundation material where the necessity of the foundation agent is described in the above was prepared, and this foundation material was used to carry out treatment using the hair volume increasing agent and the film coating agent of the present invention without using the foundation agent of the present invention. Comparative Example 2 is a comparative example in the case where a copolymer of vinyl acetate and crotonic acid (non-neutralized) which is a hydrophilic was used, a foundation material made of a resin having an appropriate concentration to be used for a foundation material where the necessity of the foundation agent is described in the above was prepared, and this foundation material was used and treatment was carried out using the hair volume increasing agent and the film coating agent of the present invention without using the foundation agent of the present invention. In the case of Comparative Example 1, the effects of raising hair was slightly exhibited when hair was washed only with hot water, but the effects were completely lost after shampooing hair, and thus, the effects of raising hair were lost as a result of shampooing while the effects of the hair volume increasing agent remained, exhibiting conventional effects of increasing hair volume. In the case of Comparative Example 2, the effects of raising hair were completely lost after washing hair with warm water and though the effects of the hair volume increasing agent remained, the effects of increasing hair volume were only conventional. Comparative Example 3 is a comparative example where treatment was carried out with only the hair volume increasing agent and the film coating agent of the present invention without using the foundation material and the foundation agent at all. As shown in Comparative Example 3, only the conventional effects of increasing hair volume where exhibited from the beginning. Comparative Example 4 is a comparative example in the case where a foundation agent having an amount of resin which provides a sufficient concentration was used and the waterproof resin of the present invention was not used and instead a hydrophilic resin (neutralized polymer of carboxyl modified vinyl acetate) was used, and thus, treatment was carried out using the hair volume increasing agent and the film coating agent of the present invention after this hydrophilic foundation agent was used in order to examine the effects of the foundation agent in the case where the foundation agent was prepared in the above described manner. Even though slight effects of raising hair seemed to be gained due to a large amount of resin after washing hair with warm water once, the effects were completely lost after washing hair with warm water two times, and the effects of increasing hair volume was gained only as conventional effects of increasing hair volume. Comparative Example 5 is a comparative example in the case where the waterproof resin of the present invention was not used as the hair volume increasing agent, but instead a hydrophilic resin (neutralized copolymer liquid of alkyl ester acrylate, diacetone acrylamide and methacrylic acid) was used to prepare a hair volume increasing agent and treatment was carried out using the foundation agent and the film coating agent of the present invention using this hair volume increasing agent. Though the effects of raising hair was gained, the effects of increasing hair volume after washing hair with warm water were conventional and the effects of increasing hair volume became poor after shampooing hair. That is to say it can be clearly seen from Tables 1 and 2 that the implementation of the present invention was a method for increasing hair volume having excellent effects of increasing hair volume.

### Example 6

A liquid component agent having the following composition was prepared.

| hair volume increasing agent II (liquid hair volume increasing component agent) | |
|---|---|
| polymer of carboxyl modified vinyl acetate (non-neutralized) | 23.6 wt % |
| coloring ion oxide (black) | 5.0 wt % |
| ultramicroscopic particles of silica anhydride | 0.5 wt % |
| titanium oxide | 0.1 wt % |
| ethanol anhydride | 70.8 wt % |
| total | 100.0 wt % |

The above described liquid hair volume increasing component agent was put into a pressure-resistant container, and then, a valve was installed and engaged, and after that, dimethyl ether was put through the valve under pressure so that the container was filled in with the liquid hair volume increasing component agent and dimethyl ether in a ratio of 33 wt % to 67 wt %, and thus, an aerosol product was gained. This was used as a hair volume increasing agent aerosol. The above described hair volume increasing agent aerosol was sprayed over all of the hair on a head having a crew cut hairstyle so that the hair volume increasing agent aerosol was applied to the surface of each hair of the user who desired to make hair thick, and after that, hair was loosened with a comb or a brush until the strands of hair, to which the hair volume increasing agent had been applied, were separated from each other, and the hair volume increasing agent solidified making combing smooth, and thus, a hair volume increasing coating film was formed on the surface of each hair so that each hair was coated with the hair volume increasing agent in sheath form.

Subsequently, the above described hair volume increasing agent aerosol was sprayed onto the hair where the above described hair volume increasing films had been formed in order to further increase the effects of increasing hair volume so that the hair volume increasing agent was applied to the hair, and hair was loosened with a comb or a brush until the strands of hair, to which the hair volume increasing agent had been applied, were separated from each other, and the hair volume increasing agent solidified, making combing smooth, and thus, a hair volume increasing coating film was formed on the surface of each hair so that each hair was coated with the hair volume increasing agent in sheath form, and the above described hair volume increasing film forming operation was repeated three times until a desired amount of hair volume increase was achieved. As a result, a new crew cut hairstyle where strands of hair that had clearly increased in thickness were conspicuous was created.

### Example 7

A liquid component agent having the following composition was prepared.

| hair volume increasing agent II (liquid hair volume increasing component agent) | |
|---|---|
| polymer of carboxyl modified vinyl acetate (non-neutralized) | 23.6 wt % |
| coloring ion oxide (black) | 5.0 wt % |
| ultramicroscopic particles of silica anhydride | 0.5 wt % |
| titanium oxide | 0.1 wt % |
| ethanol anhydride | 70.8 wt % |
| total | 100.0 wt % |

The above described liquid hair volume increasing component agent was put into a pressure-resistant container, and then, a valve was installed and engaged, and after that, dimethyl ether was put through the valve under pressure so that the container was filled in with the liquid hair volume increasing componentagent and dimethyl ether in a ratio of 33 wt % to 67 wt %, and thus, an aerosol product was gained. This was used as a hair volume increasing agent aerosol.

| film coating agent II (liquid film coating component agent) | |
|---|---|
| polymer of carboxyl modified | 11.0 wt % |

| vinyl acetate (non-neutralized) | |
|---|---|
| copolymer liquid of alkyl ester acrylate, diacetone acrylamide and methacrylic acid (non-neutralized) | 1.5 wt% |
| silicone (low molecular dimethyl silicon) | 0.5 wt % |
| ethanol anhydride | 87.0 wt % |
| total | 100.0 wt % |

The above described liquid film coating component agent was put into a pressure-resistant container, and after that, a valve was installed and engaged, and then, a mixed spraying agent where dimethyl ether and a liquefied petroleum gas were mixed in a ratio of 90 wt % to 10 wt % was put through the valve under pressure so that the container was filled in with the spraying agent in a ratio of 30 wt % of the liquid film forming component agent to 70 wt % of the spraying agent, and thus, an aerosol product was prepared. This was used as a film coating agent aerosol.

All the hair of a person whose hair is thinner than that of the other people by nature was divided into several places for treatment over the entirety of the head, and sequentially, the above described hair volume increasing agent aerosol was sprayed onto the surface of each hair so as to be applied to the hair, and the hair volume increasing agent was applied to all the hair while dividing the hair in the left, right, front and rear directions with the hand which did not hold the hair volume increasing agent aerosol and paying attention so that the hand was not sprayed with the hair volume increasing agent aerosol, and after that hair was loosened with a comb or a brush until the strands of hair, to which the hair volume increasing agent was applied, were separated from each other, and the hair volume increasing agent solidified, making combing smooth, and thus, a hair volume increasing coating film was formed on the surface of each hair so that each hair was coated with the hair volume increasing agent in sheath form. After making sure that any untreated portions did not remain as a precaution, the above described hair volume increasing agent in aerosol was subsequently sprayed onto the hair on the head portion where the above described hair volume increasing films had been formed in order to further increase the effects of increasing hair volume so that the hair volume increasing agent was applied to the hair, and hair was loosened with a comb or a brush until the strands of hair, to which the hair volume increasing agent had been applied, were separated from each other, and the hair volume increasing agent solidified, making combing smooth, and thus, a hair volume increasing coating film was formed on the surface of each hair so that each hair was coated with the hair volume increasing agent in cylindrical form, and the above described hair volume increasing film forming operation was repeated three times until a desired amount of hair volume increase was achieved.

A film coating agent aerosol was sprayed onto all the hair of which the volume had been increased by a desired amount through the coating of the hair volume increasing agent in sheath form on the surface of each hair as a result of the above described process for repeating the hair volume coating film forming operation, so that the film coating agent aerosol was applied to the hair after the hair volume increasing coating film had been formed, the film coating agent was applied to all the hair while dividing the hair in the left, right, front and rear directions with the hand which did not hold the film coating agent aerosol and paying attention so that the hand was not sprayed with the film coating agent aerosol, and after that, hair was loosened with a comb or a brush until the strands of hair, to which the film coating agent was applied, were separated from each other, and the film coating agent solidified, making combing smooth, and thus, a coating film was formed on the surface of each hair so that each hair was finally coated with the film coating agent in sheath form, and then, the film coating agent was fixed to and stabilized on the hair.

### Example 8

This is an example where a final coating with photo curing resin is applied in order to further increase resistance to alkali, resistance to oil and resistance to weather after using a hair volume increasing agent and a waterproof film coating agent.

| agent A (hair volume increasing agent) | |
|---|---|
| copolymer liquid of N-methacryloyloxyethyl N, N-dimethyl ammonium-α-N-methyl carboxybetaine and alkyl ester methacrylate containing 10wt% carbon black | 20.7 wt % |
| silicone | 1.2 wt % |
| sorbitan sesquioleate | 1.2 wt % |
| talc | 34.8 wt % |
| ethyl alcohol | 42.1 wt % |
| total | 100 wt % |

| agent B (waterproof film coating agent) | |
|---|---|
| copolymer of crotonic acid, vinyl acetate and vinyl neodecanoate (non-neutralized) | 10.0 wt % |
| silicone | 5.0 wt % |
| ethyl alcohol | 85.0 wt % |
| total | 100.0 wt % |

| agent C (photo polymerizing film coating agent) | |
|---|---|
| bifunctional acrylate | 20.0 wt % |
| filler, dispersing agent other (initiator, reducer, | 80.0 wt % |
| stabilizer) | minuscule amount |
| total | 100.0 wt % |

A pressure-resistant container was filled with 65 wt % of agent A (hair volume increasing agent) and 35 wt % of liquefied petroleum gas, and thus, an aerosol product was provided as an agent A spray (hair volume increasing agent aerosol). In addition, a pressure-resistant container was filled with 55 wt % of agent B (waterproof film coating agent) and 45 wt % of a spraying agent made of 50 wt % of liquefied petroleum gas and 50 wt % of dimethyl ether, and thus, an aerosol product was provided as an agent B spray (waterproof film coating agent aerosol).

An appropriate amount of the above described agent A spray was applied to hair until effects of increasing hair volume were gained on thin hair. After that, the hair was lightly loosened with a comb, and the hair was dried while being loosened with a comb for a while until the surface became dry in such a manner that the surface had no adhesiveness, and thus, hair was coated with a hair volume increasing agent in sheath form. When the hair was sufficiently dry, it was combed to the front and the agent B spray was thoroughly applied, so that the hair was coated with the agent, and after that, the hair was sufficiently loosened with a comb. The hair was dried while being loosened with a comb for a while until the surface became dry in such a manner that the strands of hair did not entangle or adhere to each other when the hair was dried, and the surface had no adhesiveness. When the hair was sufficiently dried, it was combed back, and then, the agent B spray was again thoroughly applied to the hair, so that the hair was coated with the agent. This operation was carried out in the same manner in a state where hair was combed to the left and right, and after that, the hair was dried while being loosened with a comb for a while until the surface became dry in such a manner that the surface had no adhesiveness, and then, the hair was coated with the waterproof film coating agent in sheath form. After that, the hair was dried using a dryer. When the hair was sufficiently dry, it was lightly washed with warm water, so that the water soluble components and pieces of resin created during the process were washed away, and after that, the hair was dried using a dryer.

Subsequently, the hair was combed to the front, and after that, drops of agent C placed in a small bottle container was applied to the hair little by little with care, so that the agent was applied and spread on the hair, and thoroughly applied so that the hair was completely coated with the agent using a comb or a brush, and after that, the hair was thoroughly loosened with a comb. The hair was dried while being loosened with a comb for a while until the surface became dry to such a degree that strands of hair did not entangle or adhere to each other and the surface had no adhesiveness. The hair was again combed back, and the agent C placed in a small bottle container was dropped little by little onto the surface of each hair, and the agent was applied and spread on the hair, so that the hair was thoroughly coated with the agent using a comb or a brush, and then, the agent C spread on the surface of each hair and was dried. The hair was dried while being loosened with a comb for a while until the hair became of a state where strands of hair did not adhere to each other and the surface was almost dry. This operation was carried out in the same manner in a state where the hair was combed to the left and right, and the hair was coated with a photo polymerizing film coating agent in sheath form. After that, the hair was irradiated with visible light having a wavelength of 410 nm to 500 nm for approximately 15 seconds while being loosened with a comb, and after that, the hair was left for 5 minutes so as to be sufficiently dried, and then, the hair was lightly washed, so that the water soluble components were washed away, and after that, the hair was set as desired while using a dryer.

### Example 9

This is an example where hair is coated with a photo curing resin which is a waterproof film coating agent having alkali resistant properties, oil resistant properties and weather resistant properties after using a foundation agent and a waterproof hair volume increasing agent.

| agent D (foundation agent) | |
|---|---|
| carboxyl modified vinyl acetate polymer (non-neutralized) | 7.0 wt % |
| copolymer of vinyl acetate and crotonic acid | 8.0 wt % |
| (non-neutralized) | |
| silicone (low molecular dimethyl silicon) | 0.2 wt % |
| higher aliphatic acid | 0.3 wt % |
| ethanol anhydride | 84.5 wt % |
| total | 100.0 wt % |

The above described foundation agent was divided into polyethylene containers, and this was provided as agent D (foundation agent).

| agent E (hair volume increasing agent) | |
|---|---|
| carboxyl modified vinyl acetate polymer (non-neutralized) | 23.6 wt % |
| coloring iron oxide (black) | 5.0 wt % |
| ultramicroscopic particles of silica anhydride | 0.5 wt % |
| titanium oxide | 0.1 wt % |
| ethanol anhydride | 70.8 wt % |
| total | 100.0 wt % |

The above described agent E (hair volume increasing agent) was put into a pressure-resistant container, and after that, a valve was installed and engaged, and then, dimethyl ether was passed through the valve, so that the pressure-resistant container was filled under pressure, and thus, an aerosol product was gained of which the ratio became such that agent E was 33 wt % and dimethyl ether was 67 wt %. This was provided as agent E spray (hair volume increasing agent aerosol).

| agent F (photo polymerizing film coating agent) | |
|---|---|
| bifunctional acrylate | 25.0 wt % |
| filler, dispersing agent | 75.0 wt % |
| others (initiator, reducer, stabilizer) | minuscule amount |
| total | 100.0 wt % |

An amount sufficient to soak the hair of the above described agent D that was divided into plastic containers was applied to all of the hair on the portion of the head where hair grows and where it is desired for the thickness to increase, and the foundation agent was applied to the hair using a small brush, and the hair was loosened with a comb or a brush, or by rubbing the hair together with the hands, until strands of hair to which the agent D had been applied were separated from each other and the agent D solidified, making combing smooth. A coating film was formed in such a manner that the surface of each hair was coated with the agent D in sheath form starting from the vicinity of the hair root toward the tip of the hair, and thus, the foundation agent for hair was fixed to and stabilized on the hair before the hair was thickened. The above described operation was repeated two times for each small area on the portion of a head where hair grows and hair is desired to be thickened, until the agent sufficiently spread on all of the hair in the area.

Next, the hair was combed to the front and the agent E spray was thoroughly applied so that the hair was coated with the agent, and after that, the hair was thoroughly loosened with a comb. The hair was dried while being loosened with a comb for a while until the surface became dry to such a degree that the surface had no adhesiveness. When the hair was sufficiently dry, it was combed back, and then, the agent E spray was again thoroughly applied, so that the hair was coated with the agent. This operation was carried out in the same manner in a state where the hair was combed to the left and right, and thus, an appropriate amount of agent was applied to the hair until effects of increasing hair volume were gained. After that, the hair was lightly loosened with a comb. The hair was dried while being loosened with a comb for a while until the surface became dry to such a degree that the surface had no adhesiveness, and thus, the hair was coated with the hair volume increasing agent in sheath form. After that, the hair was dried using a dryer. When the hair was sufficiently dry, it was lightly washed, so that the water soluble components and pieces of resin which were created during the process were washed away, and after that, the hair was dried using a dryer.

Subsequently, the hair was combed to the front, and after that, drops of the agent F were applied little by little to the coating film of the hair volume increasing agent component from a small bottle container, and after that, the agent was applied with care to the hair and spread over the hair using a comb or a brush, so that the hair was thoroughly coated with the agent, and after that, the hair was thoroughly loosened with a comb and dried while being loosened for a while, so that strands of hair did not entangle or adhere to each other. The hair was again combed back, and drops of the agent F were applied little by little to the hair from a small bottle container, so that the surface of each hair (on the coating film of the hair volume increasing agent component) was coated with care with the agent which spread, and thus, the agent F was thoroughly applied, so that the hair was coated with the agent using a comb or a brush so as to spread over the entirety of the surface of each hair, and after that, the hair was dried while being loosened with a comb for a while until the surface became dry to such a degree that the surface had no adhesiveness. This operation was carried out in the same manner in a state where the hair was combed to the left and right, so that the agent was thoroughly applied so as to spread on the entirety of the surface of each hair. After that, the hair was lightly loosened with a comb to such a degree that the surface had no adhesiveness and no drippings were created on the surface, and thus, the hair was loosened with a comb for a while so as to be dried, and thus, the hair was coated with a photo polymerizing film coating agent in sheath form. After that, the hair was irradiated with visible light having a wavelength of 410 nm to 500 nm for approximately 15 seconds while the hair was being loosened with a comb, and after that, the hair was left for 5 minutes so as to be sufficiently dried, and then, the hair was lightly washed, so that the water soluble components were washed away, and after that, the hair was set as desired using a dryer.

### Example 10

| agent J (hair volume increasing agent) | |
|---|---|
| copolymer liquid of N-methacryloyloxyethyl N, N-dimethyl ammonium-α-N-methyl carboxybetaine and alkyl ester methacrylate containing 10 wt % of carbon black | 20.7 wt % |
| silicone | 1.2 wt % |
| sorbitan sesquioleate | 1.2 wt % |
| talc | 34.8 wt % |
| ethyl alcohol | 42.1 wt % |
| total | 100.0 wt % |

| agent K (waterproof film coating agent) | |
|---|---|
| copolymer of crotonic acid, vinyl acetate and vinyl neodecanoate (non-neutralized) | 11.0 wt % |
| silicone | 5.0 wt % |
| ethyl alcohol | 84.0 wt % |
| total | 100.0 wt % |

| agent L (oil resistant and alkali resistant film coating agent) | |
|---|---|
| nitrocellulose | 6.0 wt % |
| silicone | 0.3 wt % |
| ester based plasticizing agent (CTEA) | 0.5 wt % |
| acetone | 92.2 wt % |
| total | 100.0 wt % |

A pressure-resistant container was filled with 65 wt % of agent J (hair volume increasing agent) and 35 wt % of liquefied petroleum gas so as to be provided as an aerosol product used as an agent J spray (hair volume increasing agent aerosol). In addition, a pressure-resistant container was filled with 55 wt % of agent K (waterproof film coating agent) and 45 wt % of a spraying agent made of 50 wt % of liquefied petroleum gas and 50 wt % of dimethyl ether so as to be provided as an aerosol product used as an agent K spray (waterproof film coating agent aerosol).
An appropriate amount of the above described agent J spray was applied to thin hair until effects of increasing hair volume were gained. After that, the hair was lightly loosened with a comb, and the hair was loosened with a comb for a while until the surface became dry to such a degree that the surface had no adhesiveness, and thus, the hair was coated with the hair volume increasing agent in sheath form. When the hair was sufficiently dry, it was combed to the front and the agent K spray was thoroughly applied so that the hair was coated with the agent, and after that, the hair was thoroughly loosened with a comb. The hair was loosened with a comb for a while and dried until the surface became dry to such a degree that strands of hair did not entangle and adhere to each other when the hair was dried, and the surface had no adhesiveness. When the hair was sufficiently dry, it was combed back, and then, the agent K spray was again thoroughly applied, so that the hair was coated with the agent. This operation was carried out in the same manner in a state where the hair was combed to the left and right, and after that, the hair was dried while being loosened with a comb for a while until the surface became dry to such a degree that the surface had no adhesiveness, and then, the hair was dried using a dryer. When the hair was sufficiently dry, it was washed with warm water, so that the water soluble components and pieces of resin which were created during the process were washed away, and after that, the hair was dried using a dryer.
Subsequently, the hair was combed to the front, and after that, drops of agent L were applied little by little to the hair from a small bottle container, and thus, the agent was applied to the hair with care and spread, so that the agent was thoroughly applied in such a manner that the hair was coated with the agent using a comb or a brush, and after that, the hair was thoroughly loosened with a comb and dried while being loosened with a comb for a while until the surface became dry to such a degree that strands of hair did not entangle or adhere to each other and the surface had no adhesiveness. The hair was again combed back while the agent L was dropped onto the hair little by little from the small bottle container so as to be applied to the surface of each hair and spread, and after that, the agent L was thoroughly applied, so that the hair was coated with the agent using a comb or a brush, and the agent L spread on the surface of each hair, and the hair was dried. The hair was dried while being loosened with a comb for a while until the surface became of an almost dry state, where strands of hair did not adhere to each other. This operation was carried out in the same manner in a state where the hair was combed to the left and right, so that the outermost coating film of each hair was coated with an oil resistant and alkali resistant film coating agent in sheath form. The hair was loosened with a comb for a while until the surface had no adhesiveness and there were no drippings on the surface of the hair. Subsequently, the room was sufficiently ventilated, and then the hair was sufficiently dried using a dryer. Finally, the hair was lightly washed so that the water soluble components were washed away, and after that, the hair was set as desired using a dryer.

### Industrial Applicability

In accordance with the "method for increasing hair volume" according to the present invention, a large amount of hair on top of the scalp can easily be treated without failure, and in addition, the method can be implemented in a short period of time and can be provided at a low cost, and therefore, it is certain that the method will be implemented in full scale at barbers and beauty salons, and economic effects can also be expected.

## Claims

1. A method for increasing hair volume, **characterized in that** a hair volume increasing coating film with a finish in cylindrical or sheath form is formed on hair when a waterproof hair volume increasing agent is applied, fixed and stabilized on the surface of each hair in a portion where it is desired for the hair to increase in thickness.

2. A method for increasing hair volume, **characterized in that** a coating film with a finish in cylindrical or sheath form is formed on hair when a hair volume increasing agent is applied, fixed and stabilized on the surface of each hair in a portion where it is desired for the hair to increase in thickness so that a hair volume increasing coating film is formed, and a waterproof film coating agent is applied, fixed and stabilized on the surface of each hair on which said hair volume increasing coating film is formed.

3. The method for increasing hair volume according to the above described claim 2, wherein a waterproof hair volume increasing agent is used as the hair volume increasing agent.

4. The method for increasing hair volume according to the above described claim 1 or 3, wherein the waterproof hair volume increasing agent for forming a waterproof hair volume increasing coating film is selected from among single, mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate, urethane modified resins, silicone modified resins.

5. The method for increasing hair volume according to the above described claim 1, **characterized in that** a waterproof hair volume increasing agent in liquid form is applied on the surface of each hair, and after that, the applied waterproof hair volume increasing agent is fixed and dried while the hair is loosened using a comb or a brush, and thereby, a hair volume increasing coating film in cylindrical or sheath form is provided on each hair.

6. The method for increasing hair volume according to any of the above described claim 2 to 5, **characterized in that** a waterproof film coating agent in liquid form is applied on the surface of each hair on which a hair volume increasing film is fixed and stabilized, and after that, the applied film coating agent is fixed and dried while the hair is loosened using a comb or a brush, and thereby, a waterproof coating film in cylindrical or sheath form is provided on each hair.

7. The method for increasing hair volume according to the above described claim 4 or 6, **characterized in that** the waterproof film coating agent is an acid-proof, alkaline-proof and waterproof resin or a photo-setting resin.

8. The method for increasing hair volume according to any of the above described claim 2 to 6, wherein the waterproof film coating agent is selected from among nitrocellulose, latexes which become a waterproof coating film after curing from among multipurpose latexes, such as NR or other general diene based synthetic polymers, polymer latexes, including MBR, BR, VP, NIR, SIR, IR, FKM, EPM and CSM, or single, mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate, urethane modified resins, silicone modified resins.

9. The method for increasing hair volume according to any of the above described claim 1 to 8, **characterized in that** the resin of the hair volume increasing agent made of a waterproof resin in liquid form or the resin of the film coating agent made of a waterproof resin in liquid form is a waterproof and cleaning proof type resin having cleaning proof properties such that the coating film cannot be easily washed off with an acid or a neutralizing washing agent for hair after being dried in a non-neutralized state, and the waterproof and cleaning proof properties of the coating film can be reversed with an alkali neutralizing agent.

10. The method for increasing hair volume according to any of the above described claim 1, 2, 3, 5, 6, 7 and 9, wherein the resin of the hair volume increasing agent made of a waterproof resin in liquid form or the resin of the film coating agent made of a waterproof resin in liquid form is a waterproof and cleaning proof type resin having cleaning proof properties such that the coating film cannot be easily washed off with an acid or a neutralizing washing agent for hair after being dried in a non-neutralized state, and the waterproof and cleaning proof properties of the coating film can be reversed with an alkali neutralizing agent, and the resin is any of non-neutralizing copolymers of octyl amide acrylate and ester acrylate, non-neutralizing copolymers of alkyl ester acrylate, diacetone acrylamide and methacrylic acid, non-neutralizing copolymers of alkyl ester acrylate, alkyl ester methacrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl amide acrylate, hydroxypropyl acrylate and butyl amino ester methacrylate, non-neutralizing polymers of carboxyl modified vinyl acetate.

11. The method for increasing hair volume according to any of the above described claim 1 to 10, **characterized in that** a foundation agent for hair is applied, fixed and stabilized on the hair before a hair volume increasing coating film is formed on the hair.

12. A method for fixing and stabilizing a foundation agent for hair on hair before the hair is thickened, **characterized in that** a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied to all of the hair, and the hair is loosened with a comb or a brush until strands of hair to which the foundation agent for hair has been applied are separated from each other, the foundation agent for hair solidifies, making combing smooth, and thereby, a coating film that is compatible with hair coated with a foundation agent for hair in cylindrical or sheath form, has flexibility and is highly waterproof is formed on the surface of each hair.

13. The method for fixing and stabilizing a foundation agent for hair according to the above described claim 12, wherein one component of the foundation for hair in liquid form that is compatible with hair, has flexibility, has little malleability and is highly waterproof is selected from single, mixture or copolymers of the followings: non-neutralizing copolymers of octyl amide acrylate and ester acrylate, non-neutralizing copolymers of alkyl ester acrylate, diacetone acrylamide and methacrylic acid, non-neutralizing copolymers of vinyl acetate and crotonic acid, non-neutralizing copolymers of crotonic acid, vinyl acetate and vinyl neodecanoate, non-neutralizing copolymers of octyl acrylamide acrylate, hydroxypropyl acrylate and butyl amino ethyl methacrylate, non-neutralizing polymers of carboxyl modified vinyl acetate, urethane resins, silicone resins, urethane modified resins, silicone modified resins.

14. A method for increasing hair volume, **characterized by** comprising: the step of fixing and stabilizing a foundation agent for hair on hair before the hair is thickened, where a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied on the surface of the hair, and the hair is loosened with a comb or a brush until strands of hair to which the foundation agent for hair has been applied are separated from each other, the foundation agent solidifies, making combing smooth, and thereby, a coating film that is compatible with hair coated with a foundation agent for hair in cylindrical or sheath form, has flexibility and is highly waterproof is formed on the surface of each hair; and the step of repeating a hair volume increasing coating film forming operation until a desired amount of hair increase is gained, wherein in said hair volume increasing coating film forming operation, a hair volume increasing agent in liquid form for forming a hair volume increasing coating film having a large volume that has appropriate affinity and adhesiveness with a waterproof coating film is applied on the surface of the hair of the hair portion where said waterproof coating film is formed in such a manner that the surface of each hair is coated with a foundation agent for hair in cylindrical or sheath form in said step of fixing and stabilizing a foundation agent for hair on hair, hair is rubbed together and/or loosened using a comb or a brush so that the hair volume increasing agent is applied to the hair, and the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a foundation agent and a hair volume increasing agent in cylindrical or sheath form, and thus, the hair volume increasing agent is fixed and stabilized on the hair together with the foundation agent, said hair volume increasing agent in liquid form is applied on the surface of the hair on the hair portion where said hair volume increasing coating film is formed in order to further enhance the effects of increasing hair volume, the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a hair volume increasing agent in cylindrical or sheath form.

15. A method for increasing hair volume, **characterized by** comprising: the step of fixing and stabilizing a foundation agent for hair on hair before the hair is thickened, where a sufficient amount of a foundation agent for hair in liquid form which forms a coating film that is compatible with hair, has flexibility, has little malleability and is highly waterproof for wetting the hair is applied to the surface of hair on the portion of a head where hair grows, where it is desired for the hair to increase in thickness, hair is rubbed together and/or loosened using a comb or a brush so that the foundation agent for hair is applied on the surface of the hair, and the hair is loosened with a comb or a brush until strands of hair to which the foundation agent for hair has been applied are separated from each other, the foundation agent solidifies, making combing smooth, and thereby, a coating film that is compatible with hair coated with a foundation agent for hair in cylindrical or sheath form, has flexibility and is highly waterproof is formed on the surface of each hair; the step of repeating a hair volume increasing coating film forming operation until a desired amount of hair increase is gained, wherein in said hair volume increasing coating film forming operation, a hair volume increasing agent in liquid form for forming a hair volume increasing coating film having a large volume that has appropriate affinity and adhesiveness with a waterproof coating film is applied on the surface of the hair of the hair portion where said waterproof coating film is formed in such a manner that the surface of each hair is coated with a foundation agent for hair in cylindrical or sheath form in said step of fixing and stabilizing a foundation agent for hair on hair, hair is rubbed together and/or loosened using a comb or a brush so that the hair volume increasing agent is applied to the hair, and the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a foundation agent and a hair volume increasing agent in cylindrical or sheath form, and thus, the hair volume increasing agent is fixed and stabilized on the hair, said hair volume increasing agent in liquid form is applied on the surface of the hair on the hair portion where said hair volume increasing coating film is formed in order to further enhance the effects of increasing hair volume, the hair is loosened with a comb or a brush until strands of hair to which the hair volume increasing agent has been applied are separated from each other, the hair volume increasing agent solidifies, making combing smooth, and thereby, a hair volume increasing coating film is formed on the surface of each hair coated with a hair volume increasing agent in cylindrical or sheath form; and the step of fixing and stabilizing a film coating agent on hair, wherein a film coating agent for forming a waterproof coating film having appropriate affinity and adhesiveness with said hair volume increasing coating film is applied to the surface of the hair which has been increased by a desired volume in such a manner that the surface of each hair is coated with a hair volume increasing agent together with a foundation agent in cylindrical or sheath form in the step where said hair volume increasing coating film forming operation is repeated and the hair is loosened with a comb or a brush until the strands of hair to which the film coating agent has been applied are separated from each other and the film coating agent solidifies, making combing smooth, and thereby, a film coating is formed on the surface of each hair which is coated with a film coating agent in cylindrical or sheath form.

16. A hair volume increasing agent set used for a method for increasing hair volume, comprising: a foundation agent for hair in liquid form made of a lower alcohol solution containing 3 wt % to 35 wt % of a resin component for forming a coating film which is compatible with hair, has flexibility, has little malleability and is highly waterproof; a hair volume increasing agent in liquid form made of a lower alcohol solution containing 95 wt % to 5 wt % of a resin composition for forming a coating film which has appropriate affinity and adhesiveness with a coating film formed on hair of said foundation agent for hair, increases the volume of hair to a great degree, has flexibility, has little malleability and is highly waterproof; and a film coating agent made of a resin component for forming a coating film which has appropriate affinity and adhesiveness with a hair volume increasing coating film that is formed on hair of said hair volume increasing agent, preserves the effects of increasing hair volume and is highly waterproof.
